Europäisches Patentamt

(19) European Patent Office

Office européen des brevets

(11) EP 0 606 646 B1

(12) **EUROPEAN PATENT SPECIFICATION**

(45) Date of publication and mention
of the grant of the patent:
**24.09.1997 Bulletin 1997/39**

(51) Int. Cl.⁶: **A61M 1/36**, B01D 39/16,
A61K 35/14

(21) Application number: **93120963.9**

(22) Date of filing: **27.12.1993**

(54) **Filtermaterial, apparatus and method for removing leukocytes**

Filtermaterial, Vorrichtung und Verfahren zum Abtrennen von Leukozyten

Matériau filtrant, dispositif et méthode pour la séparation de leucocytes

(84) Designated Contracting States:
**DE FR GB IT NL**

(30) Priority: **28.12.1992 JP 358599/92**

(43) Date of publication of application:
**20.07.1994 Bulletin 1994/29**

(73) Proprietor: **ASAHI MEDICAL CO., LTD.**
**Chiyoda-Ku, Tokyo 100 (JP)**

(72) Inventors:
• **Shyuichiro, Inadome**
**Oita-shi, Oita-ken (JP)**

• **Shin-Ichiroh, Oka**
**Oita-shi, Oita-ken (JP)**

(74) Representative: **Strehl Schübel-**
**Hopf Groening & Partner**
**Maximilianstrasse 54**
**80538 München (DE)**

(56) References cited:
**EP-A- 0 267 286**    **EP-A- 0 313 348**
**EP-A- 0 329 303**    **EP-A- 0 500 472**
**EP-A- 0 554 460**

Printed by Rank Xerox (UK) Business Services
2.14.14/3.4

EP 0 606 646 B1

**Description**

BACKGROUND OF THE INVENTION

FIELD OF THE INVENTION

The present invention relates to a filter material for removing leukocytes from a whole blood product or a red cell product. More particularly, the present invention is concerned with a filter material for removing leukocytes from a whole blood product or a red cell product, comprising a porous element, and wherein the porous element has, in a surface portion thereof, basic functional groups at a specific density and nonionic hydrophilic groups in a specific amount relative to the amount of the basic functional groups, thereby imparting the filter material with a high leukocyte-removing capability, i.e., the ability to reduce the amount of leukocytes in the blood product, when treated with the filter material, to a level as low as $10^{-4}$ to $10^{-6}$ or less in terms of the leukocyte residual ratio. The present invention is also concerned with a leukocyte-removing apparatus having the above-mentioned filter material accommodated therein. The present invention is further concerned with a method for efficiently removing leukocytes from a whole blood product or a red cell product.

DISCUSSION OF RELATED ART

In recent years, in accordance with the progress of immunology and transfusion medicine, a blood component transfusion, in which blood components required to treat a particular disease are transfused, has been increasingly carried out in stead of the conventional whole blood transfusion. The blood component transfusion is a therapy which is desirable because the burden on a patient receiving transfusion can be alleviated, and because it ensures a high treatment efficacy. Various types of blood products, such as concentrated red cells (CRC), or packed red cells (PRC), platelet concentrate (PC) and platelet-poor plasma (PPP) etc. are employed in the blood component transfusion, and are prepared by subjecting donated whole blood to centrifugation. It has become apparent, however, that blood products obtained by centrifugation separation of a whole blood product contain leukocytes in high concentration, and that many kinds of side effects of transfusion are caused by the contained leukocytes. Side effects of transfusion include not only relatively mild side effects, such as headache, nausea, chilliness and nonhemolytic febrile transfusion reactions, but also serious side effects, such as allosensitization, post-transfusion GVHD (graft versus host diseases), in which transfused leukocytes attack the skin and internal organs of the transfusion recipient, transmission of viruses present in the leukocytes, e.g., cytomegalovirus transmission. The above-mentioned side effects of transfusion reactions can be effectively prevented by removing the leukocytes contained in the blood products.

Generally, blood products for use in transfusion, such as a whole blood product and a red cell product, contain $10^{7}$ leukocytes per ml. It is recognized that the number of leukocytes injected into a recipient at one transfusion must be limited to about 100,000,000 or less for avoiding relatively mild side effects, such as headache, nausea, chilliness and febrile reactions. To meet this requirement, leukocytes must be removed from a blood product to a level of $10^{-1}$ to $10^{-2}$ or less in terms of a leukocyte residual ratio. For preventing allosensitization, however, leukocytes must be removed from a blood product to a level of $10^{-4}$ or less in terms of a leukocyte residual ratio. With respect to post-transfusion GVHD and viral transmission, no generally accepted standards for leukocyte-removal has been established. However, it is expected that transmission of a virus which is believed to exist only in leukocytes, such as cytomegalovirus, adult T cell leukemia virus and post-transfusion GVHD, could be prevented by removing leukocytes to a level of $10^{-4}$ to $10^{-6}$ or less in terms of a leukocyte residual ratio. Further, it is also expected that the probability of infection with a virus which is believed to exist in both leukocytes and plasma, such as HIV, can be decreased by removing leukocytes.

The methods for removing leukocytes from a blood product can generally be classified into two methods. One is a method in which leukocytes are separated by centrifugation, taking advantage of a specific gravity difference therebetween. The other is a filtering method in which leukocytes are removed by a filter material comprising a porous element, such as a fibrous fabric or a non-fibrous porous structure. In particular, a filtering method in which leukocytes are adsorption-removed by a filter material is widely employed due to the advantages of high capability to remove leukocytes, ease in handling and low cost.

The mechanism of removal of leukocytes by means of a fibrous fabric or a non-fibrous porous structure has been considered to be adsorption of leukocytes on the surface of the porous element, such as the fibrous fabric or non-fibrous porous structure through the contact of the leukocytes with the surface. Conventionally, studies for improving the leukocyte-removing ability of a filter material have been concentrated mainly on the increase of the frequency of contact between the filter material and leukocytes by way of, for example, decreasing the average fiber diameter of fibers, or increasing the packing density of the fibrous materials, or choosing a non-woven fabric having a more uniform distribution of fiber diameters [see, for example, Unexamined Japanese Patent Application Laid-Open Specification (Kokai) H2-203909]. However, with respect to blood products having a high concentration of red blood cells, such as a red cell product or a whole blood product, it is difficult to achieve the improvement in leukocyte-removing ability by only the above-mentioned ways. This is because when the frequency of contact between the filter material and leukocytes is increased by way of changing only the physical factors of the filter material, the filtration operation is likely to suffer from an increase in resistance of the filter material to the passage of the red cells (which are present in the blood product in a high concentration) therethrough, thereby disadvantageously causing not only the need of an extremely long time for blood treatment but also the hemolysis due to the destruction of a red cell membrane, so that serious problems occur. Therefore, when it is considered that the resistance of the filter material to the passage of the red cells therethrough inevitably increases as the leukocyte-removing ability is increased by choosing the above-mentioned physical factors of the filter material, there should be a limitation on a tolerable range with respect to the physical factors which can be chosen and, accordingly, there should also be a limitation with respect to the leukocyte-removing ability which can be achieved by the choice of physical properties of the filter material. Thus, when removal of leukocytes from the blood product to a level as low as $10^{-4}$ to $10^{-6}$ or less in terms of a leukocyte residual ratio is intended for preventing a blood recipient from suffering serious side effects, such as allosensitization, post-transfusion GVHD and viral transmission, it has been difficult to perform the blood treatment within a reasonably short period of time.

On the other hand, when the above-mentioned mechanism of removal of leukocytes by filter materials (i.e., adsorption of leukocytes on the surface of the filter material) is considered, it is conceivable to increase the adsorptivity of the filter material for leukocytes by imparting appropriate chemical properties to the surface of the filter material. However, in the studies of leukocyte-removing filter materials for obtaining blood products to be used in transfusion, almost no attempts have heretofore been made to increase the leukocyte removal ratio by imparting specific chemical properties to the surface of a filter material, except some attempts as disclosed in Unexamined Japanese Patent Application Laid-Open Specification (Kokai) H1-249063, WO 87/05812, EP 0 500 472 A2 and Unexamined Japanese Patent Application Laid-Open Specification (Kohyo) H3-502094 (corresponding to WO 89/03717).

Unexamined Japanese Patent Application Laid-Open Specification (Kokai) H1-249063 discloses an improved process for selectively removing leukocytes from a platelet product by the use of a filter material having hydrophilicity and negative charges in a surface portion thereof. This technique was proposed for increasing the ratio for platelets (which are known as cells having high adsorbability on the filter material) to pass the filter material while maintaining the adsorption-removal ratio of leukocytes.

WO 87/05812 also discloses a process for selectively removing leukocytes from a platelet product by the use of a filter material having, in a surface portion thereof, nitrogen-containing basic functional groups. This technique was proposed for selectively adsorption-removing leukocytes while passing platelets through the filter material without being adsorbed thereon. In this prior art reference there is a description to the effect that the material having nitrogen-containing basic functional groups in a surface portion thereof is caused to have positive charges in a physiological liquid for use in suspending cells therein, so that platelets and leukocytes, both having negative charges, are likely to be adhered to the surface of the filter material. This is substantiated by both Examples and Comparative Examples of the reference in which as the proportion of the nitrogen-containing basic functional groups in the peripheral portions of the fibers is increased, the removal ratio of leukocytes in the treatment of a fresh whole blood product with the filter material increases, so that the leukocyte residual ratio ultimately reaches a level as low as approximately $10^{-2}$. The inventors of WO 87/05812 also reported similar experimental results in a scientific paper (Advanced methods for leucocyte removal by blood filtration. In: Brozovic B. ed. The Role of Leucocyte Depletion in Blood Transfusion Practice. Oxford: Blackwell Scientific Publications, 1989:35-40). However, according to experiments conducted following the methods disclosed in WO 87/05812 and the above-mentioned paper, it has been revealed that when red cell products are treated, an improvement in removal of leukocytes cannot be obtained as expected. Actually, in the treatment of a whole blood product or a red cell product, it was impossible to reduce the leukocyte content thereof to a level as low as $10^{-4}$ to $10^{-6}$ or less in terms of a leukocyte residual ratio. Further, it should be noted that when the blood treated with the filter material of WO 87/05812 was a whole blood product or a red cell product, the leukocyte removal ratio did not increase in proportion to the amount of nitrogen-containing basic functional groups in the surface portion of the filter material, but, in some cases, the leukocyte removal ratio was rather low as compared to that obtained with respect to the filter material having no nitrogen-containing basic functional groups.

EP 0 500 472 A2 discloses a process for removing both leukocytes and platelets from a red cell product by the use of a filter material having a positive zeta potential. This technique was proposed for improving the platelet removal ratio while maintaining the ratio for the red cells to pass through the filter material, but not for improving the leukocyte removal ratio. In this connection, it should be noted that in this reference it is described to the effect that with respect to leukocytes, good removal can be achieved even without practicing the surface modification disclosed therein. Of course, in this reference there is no description about the technique for increasing the leukocyte removal ratio. In fact, no significant difference in leukocyte removal ratio is observed as between the data of Examples and the data of Comparative Examples in this reference, but it is observed that, in some cases, the leukocyte removal ratios achieved in Examples (in which use is made of filter materials having a positive zeta potential) are rather lower than those in Comparative Examples in which the positive zeta potential requirement is not satisfied. On the other hand, in this reference, it is described that leukocytes were removed from a red cell product to a level of $10^{-3.7}$ in terms of the leukocyte residual ratio by the treatment of the red cell product with a polyurethane spongy structure having thereon a coating containing a quaternary ammonium salt (Example 6 of the reference). However, in the reference, there are no working examples in which the removal of leukocytes from a whole blood product or a red cell product to a level of $10^{-4}$ to $10^{-6}$ or less in terms of the leukocyte residual ratio was realized. With respect to the materials for filter materials, the reference teaches only Egyptian cotton, a polyvinyl formal porous structure and a polyurethane porous structure, but does not describe a non-woven fabric composed synthetic fibers or any other materials and morphologies than mentioned above. It is well known that a quaternary ammonium salt has antibacterial activity. This antibacterial activity is considered to be ascribed to the activity of the salt to destroy the cell membrane of bacteria (Tomiki Ikeda et al., "Antibacterial Polycation" in a book entitled "Macromolecule and Medicine" edited by Kiichi Takemoto and published by Mita Publishing Company, 1989, pp. 459-496). Therefore, the filter material of EP 0 500 472 A2 has a danger in that it is likely to damage the red cell membrane, so that this type of filter material has problems with respect to not only efficiency but also safety.

Unexamined Japanese Patent Application Laid-Open Specification (Kohyo) H3-502094 (corresponding to WO 89/03717) discloses a filter material imparted with hydrophilicity for the purpose of rendering easy the priming of a blood filter apparatus containing the filter material. This filter material was not proposed for improving the leukocyte removal ratio. In the reference there is no description teaching or suggesting that adsorption-removal of leukocytes is increased by the use of the filter material having its surface modified according to the technique disclosed therein.

As is apparent from the above, any of the prior art references mentioned above has no concept of increasing the leukocyte removal ratio to a level of $10^{-4}$ to $10^{-6}$ or less in terms of the leukocyte residual ratio by imparting appropriate chemical properties to the surface of a filter material, and also does not contain any description about not only specific chemical properties required for increasing the leukocyte removal ratio but also facts that the leukocyte removing ratio is actually increased by the use of the techniques disclosed therein.

In fact, in conventional practices of removal of leukocytes from blood products, it has been required to use a large quantity of filter materials for achieving a high ratio of leukocyte removal. In this case, if it is desired to conduct the blood filtration at a high filtration flow rate, it is necessary to decrease the packing density of the filter materials in a filter apparatus, leading to an increase in a volume of the filter apparatus. This also leads to an increase in amount of waste blood to be discarded, inevitably causing the red cell recovery to be decreased. On the other hand, if the increase of the red cell recovery to 90 % or more is desired, it is necessary to increase the packing density of filter materials in a filter apparatus, which disadvantageously leads to a lowering of the filtration flow rate in the filtration treatment of blood products, the lowering of the filtration flow rate being markedly large with the lapse of time for filtration treatment. That is, in conventional techniques, there have been serious technical dilemma.

## SUMMARY OF THE INVENTION

The present inventors have made extensive and intensive studies with a view toward developing a filter material which is capable of removing leukocytes from a whole blood product or a red cell product to a level as low as $10^{-4}$ to $10^{-6}$ or less in terms of the leukocyte residual ratio, which is effective for preventing a recipient of blood transfusion from suffering serious side effects, such as allosensitization, post-transfusion GVHD and viral transmission, and which is also capable of achieving a red cell recovery as high as 90 % or more with a high filtration flow rate in a filtration treatment of whole blood or a red cell product.

For this purpose, the present inventors first made investigations on the possibility of increasing the leukocyte removal ratio by introducing electric charges into the surface of a filter material comprising a porous element, such as a fibrous fabric and a non-fibrous porous structure, and studied the influences of the type and amount of electric charges on the removal of leukocytes from blood products. Illustratively stated, polymerizable monomers containing various types of basic functional groups having positive charges were polymerized on the surfaces of filter materials by radiation-grafting to obtain radiation-grafted types of surface-modified filter materials. Further, various polymers obtained by polymerizing the above-mentioned monomers were coated on the surfaces of filter materials to obtain coated types of surface-modified filter materials. Using these surface-modified filter materials, experimental studies have been made on the leukocyte removal ratios thereof. As a result, it has unexpectedly been found that, despite the high content of nitrogen-containing basic functional groups in the surfaces of the filter materials, the leukocyte-removing capabilities of these surface-modified filter materials are not improved or are rather poor as compared to those of the non-modified filter materials.

In these situations, the present inventors have made further intensive studies as to what types of chemical properties are needed for the basic functional groups in the surfaces of the filter materials to exert remarkably improved leukocyte removal ratio. As a result, it has surprisingly been found that when the filter material has, in a surface portion thereof, not only basic functional groups at a specific density but also nonionic hydrophilic groups in a specific amount relative to the amount of the basic functional groups, the filter material exhibits a high leukocyte-removing capacity, i.e., the ability to reduce the amount of leukocytes in the blood product treated with the filter material to a level as low as $10^{-4}$ to $10^{-6}$ or less in terms of the leukocyte residual ratio, and also found that when specific physical requirements are satisfied the filter material can also exhibit not only a red cell recovery as high as 90 % or more but also a high filtration rate. The present invention has been completed, based on these novel findings.

Accordingly, it is an object of the present invention to provide a filter material for removing leukocytes from a whole blood product or a red cell product, which is capable of reducing the amount of leukocytes in the blood product treated with the filter material to a level as low as $10^{-4}$ to $10^{-6}$ or less in terms of the leukocyte residual ratio.

It is another object of the present invention to provide the filter material as mentioned above, which is further characterized such that it exhibits not only a red cell recovery as high as 90 % or more but also a high filtration rate.

It is still another object of the present invention to provide a filter apparatus containing the filter material as mentioned above, which can be advantageously used for treating a blood product for use in blood transfusion.

It is a further object of the present invention to provide a method of removing leukocytes from a whole blood product or a red cell product, which can be practiced using the filter apparatus as mentioned above, and which can provide a leukocyte-depleted blood product having a leukocyte residual ratio as low as $10^{-4}$ to $10^{-6}$ or less.

The foregoing and other objects, features and advantages of the present invention will be apparent from the following detailed description and appended claims.

<u>DETAILED DESCRIPTION OF THE INVENTION</u>

Essentially, according to the present invention, there is provided a filter material for removing leukocytes from a whole blood product or a red cell product, comprising at least one layer of a porous element having a three-dimensional network of continuous open pores, and wherein the porous element has, in a surface portion thereof, basic functional groups at a density of from $5 \times 10^{-5}$ to $0.1$ meq/m$^2$ and nonionic hydrophilic groups, wherein a molar ratio of the basic functional groups to the nonionic hydrophilic groups is in the range from 0.6 to 6.

In the present invention, the porous element is selected from the group consisting of a fibrous fabric and a non-fibrous porous structure.

In the filter material of the present invention, it is required that the surface of the porous element have both basic functional groups and nonionic hydrophilic groups, wherein the molar ratio of the basic functional groups to the nonionic hydrophilic groups be in the range from 0.6 to 6. When the molar ratio is smaller than 0.6, a satisfactory improvement in leukocyte removal ratio cannot be obtained. Also when the molar ratio is larger than 6, the leukocyte removal ratio cannot be satisfactorily improved although the reason therefor has not been elucidated. The molar ratio of the basic functional groups to the nonionic hydrophilic groups is preferably from 0.6 to 4.0, more preferably from 0.7 to 2.5.

Examples of basic functional groups include amino groups, such as a primary amino group, a secondary amino group, a tertiary amino group; and nitrogen-containing aromatic ring groups, such as a pyridyl group and an imidazolyl group. Among these groups, an amino group can be preferably used from a viewpoint of safety in use of the filter material as medical equipment, and a tertiary amino group is most preferred.

Examples of nonionic hydrophilic groups include a hydroxyl group, an amido group, an ethereal group, a nitro group, a nitroso group, a sulfoxide group, a sulfonyl group, a phosphoryl group, a phospho group, a carboxyamido group, a sulfonamide group, a thio acid amido group, a cyano group, a cyanato group, a thiocyanato group, an isocyanato group, an isothiocyanato group and a thio carboxylic acid group. Among these groups, groups having high hydrophilicity, such as a hydroxyl group, an amido group and an ethereal group, are preferred. Of ethereal groups, a polyethylene oxide group consisting of a chain of recurring ethylene oxide units is preferred. With respect to the polyethylene oxide chain, regardless of the number of the recurring ethylene oxide units, namely, the length of the polyethylene oxide chain, one polyethylene oxide chain is defined as one functional group. When polyethylene oxide chains having various lengths are present, a number average chain length is obtained, and a polyethylene oxide chain corresponding to the number of the ethylene oxide units in the number average chain length is defined as one functional group.

In the present invention, it is required that the density of the basic functional groups present in the surface portion of the porous element be from $5 \times 10^{-5}$ to $0.1$ meq/m$^2$. When the density of the basic functional groups is less than $10^{-5}$ meq/m$^2$, a satisfactory improvement in leukocyte removal ratio cannot be achieved even though the molar ratio of the basic functional groups to the nonionic hydrophilic groups is within the range from 0.6 to 6. On the other hand, when the density is higher than $0.1$ meq/m$^2$, red cells are likely to be damaged during the filtration of a whole blood product or a red cell product. The density of nonionic hydrophilic groups present in the surface portion of the porous element is preferably from $10^{-4}$ to $6 \times 10^{-2}$ meq/m$^2$, more preferably from $5 \times 10^{-4}$ to $4 \times 10^{-2}$ meq/m$^2$.

The molar ratio of the basic functional groups to the nonionic hydrophilic groups in the surface portion of the porous element can be measured by known techniques, such as X-ray photoelectron spectroscopy (XPS), Auger electron spectroscopy (AES), secondary ion mass spectroscopy (SIMS) and attenuated total reflection-Fourier transform infrared spectroscopy (ATR-FTIR). In measurement, a surface portion of the porous element is subjected to extraction and the extract obtained is analyzed with respect to the molar ratio, using the above-mentioned known technique or nuclear magnetic resonance (NMR).

The density of basic functional groups in the surface portion of the porous element is defined by the value obtained by the following method. First, the amount of surface basic functional groups per unit weight of the porous element is measured by any of the above-mentioned techniques. More easily, the amount of basic functional groups can be obtained by measuring the amount of adsorption of an acidic dye, such as Trypan Blue, or by acid-base titration. Next, the total pore surface area per unit weight of the porous element is measured by mercury porosimetry. The above-obtained amount of surface basic functional groups per unit weight of the porous element is divided by the total pore surface area per unit weight of the porous element to thereby obtain an amount of surface basic functional groups per unit surface area of the porous element, i.e., surface density of basic functional groups.

Measurement of a total pore surface area per unit weight of the porous element by mercury porosimetry is done as follows. A portion of the porous element is sampled, and a weight (W) of the sample is measured. With respect to this sample, a total pore surface area (A) is measured using a mercury porosimeter ("Poresizer-9320, PC 2A system, sold by Shimadzu Corporation, Japan, or equipment equivalent thereto) at a pressure of from 0.1 to 180 psia. The total pore surface area per unit weight of the porous element can be obtained by formula: A/W.

In measuring the total pore surface area per unit weight of the porous element, it is preferred to take samples from the porous element at three or more portions thereof and obtain an average value of the total pore surface areas (per unit weight of the porous element) measured with respect to the three or more samples.

The porous element of the present invention can be produced from a polymer material satisfying the requirements defined in the present invention with respect to basic functional groups and nonionic hydrophilic groups. Alternatively, the porous element in the present invention may be produced by introducing a predetermined quantity of a monomer or a polymer having basic functional groups and nonionic hydrophilic groups into the surface of a porous element, i.e., a fibrous fabric or a non-fibrous porous structure which does not contain basic functional groups and nonionic hydrophilic groups. In this case, the introduction of a monomer or a polymer can be conducted by conventional techniques, such as covalent bonding, ionic bonding, graft polymerization (e.g., radiation-graft polymerization or plasma-graft polymerization), physical adsorption, embedding, precipitation immobilization and coating. From the viewpoint of ease in production and attaining good stability of the performance of the porous element, a method in which a monomer having a basic functional group and a monomer having a nonionic hydrophilic group (or a monomer having both basic functional groups and nonionic hydrophilic groups) are directly graft-polymerized on the surface of a porous element and a method in which a polymer having both basic functional groups and nonionic hydrophilic groups is coated on the surface of a porous element, are preferred. In the case of a coating method, a polymer coated on the porous element may be subjected to crosslinking so as to prevent a coming-off of the coated polymer from the porous element.

Examples of monomers having basic functional groups and which can be introduced into the surface of a porous element by graft polymerization include (meth)acrylic acid derivatives, such as dimethylaminoethyl (meth)acrylate, diethylaminoethyl (meth)acrylate, dimethylaminopropyl (meth)acrylate, and 3-dimethylamino-2-hydroxypropyl (meth)acrylate; and styrene derivatives, such as p-dimethylaminomethylstyrene and p-diethylaminoethylstyrene; and vinyl derivatives of nitrogen-containing aromatic compounds, such as 2-vinylpyridine, 4-vinylpyridine and 4-vinylimidazole. Of these examples, from the viewpoint of availability and ease in handling in conducting graft polymerization, (meth)acrylic acid derivatives containing a tertiary amino group are preferred. Among the derivatives, dimethylaminoethyl (meth)acrylate and diethylaminoethyl (meth)acrylate are more preferred.

Examples of monomers having nonionic hydrophilic groups and which can be introduced into the surface of a porous element by graft polymerization include monomers containing a hydroxyl group, such as 2-hydroxyethyl (meth)acrylate, 2-hydroxypropyl (meth)acrylate, glycerol mono(meth)acrylate and vinyl alcohol (vinyl acetate is polymerized and then hydrolyzed); monomers containing an amido group, such as (meth)acrylamide and N-vinylpyrrolidone; and monomers containing a polyethylene oxide chain, such as methoxytriethylene glycol (meth)acrylate, methoxyoctaethylene glycol (meth)acrylate and methoxypolyethylene glycol (meth)acrylate.

Examples of monomers having both basic functional groups and nonionic hydrophilic groups include a derivative obtained by reacting a compound having a primary or secondary amino group, such as dimethyl amine, with a compound having an epoxy ring, such as glycidyl (meth)acrylate.

Examples of polymers which can be coated on a porous element for introducing basic functional groups and nonionic hydrophilic groups include a copolymer obtained by polymerizing at least one monomer having basic functional groups (hereinafter referred to as "basic monomer") and at least one monomer having nonionic hydrophilic groups (hereinafter referred to as "hydrophilic monomer") and a copolymer obtained by polymerizing at least one basic monomer, at least one hydrophilic monomer and at least one other monomer. Examples of other monomers include a monomer which is ionically neutral and has no nonionic hydrophilic group (hereinafter referred to as "neutral monomer") and a monomer having acidic functional groups (hereinafter referred to as "acidic monomer"). Further examples of polymers which can be coated on a porous element for introducing basic functional groups and nonionic hydrophilic groups include a homopolymer of a monomer having both basic functional groups and nonionic hydrophilic groups (hereinafter referred to as "basic, hydrophilic monomer"), a copolymer of at least two different types of basic, hydrophilic monomers, and a copolymer of at least one basic, hydrophilic monomer and at least one monomer selected from the group consisting of a basic monomer, a hydrophilic monomer, a neutral monomer and an acidic monomer.

Polymerization of the above-mentioned monomers can be conducted by conventional methods, such as a random copolymerization method, a block copolymerization method, an alternating copolymerization method and a method in which a part of a monomer is polymerized to thereby obtain a macromonomer and then the macromonomer is polymerized with other monomers. Of these methods, from the viewpoint of technical ease, a random copolymerization method is preferred.

Examples of neutral monomers include a monomer having a vinyl group, such as styrene or methyl (meth)acrylate, and a monomer having an acetylene group. Examples of acidic monomers include a monomer having a carboxyl group, such as methacrylic acid, a monomer having a sulfonic acid group, such as styrene sulfonate; and a monomer having a phosphoric acid group, such as mono(2-acryloyloxyethyl) acid phosphate, mono(2-methacryloyloxyethyl) acid phosphate, mono(2-acryloyloxybutyl) acid phosphate and mono(2-methacryloyloxybutyl) acid phosphate.

The coating of a porous element for introducing basic functional groups and nonionic hydrophilic groups into the surface thereof may also be conducted by using a polymer blend containing a homopolymer or copolymer of a basic monomer and a homopolymer or copolymer of a hydrophilic monomer.

Further, the introduction of basic functional groups and nonionic hydrophilic groups into the surface of a porous element may be conducted by a method in which a compound having an epoxy ring, such as polyglycidyl (meth)acrylate, is coated or grafted on a porous element and then a compound having a primary or secondary amino group, such as dimethyl amine, is reacted with the polymer already introduced on the porous element so as to introduce both basic functional groups and nonionic hydrophilic groups into the surface of the porous element.

The morphology and structure of the filter material of the present invention are not particularly limited as long as it comprises a porous element selected from the group consisting of a fibrous fabric and a non-fibrous porous structure having a three-dimensional network of continuous open pores. For example, the porous element may have the morphology and structure of any of conventional fibrous structures or non-fibrous porous structures, such as a fibrous non-woven fabric, a fibrous woven fabric, paper, a mesh, a spongy structure and a porous membrane. Of these, the morphology and structure of a non-woven fabric is preferred. A non-woven fabric can be produced by conventional techniques, such as the melt blow method, the flash spinning method and the spun lace method. The terminology "non-woven fabric" used herein defines a cloth-form fabric in which a mass of fibers or yarns are chemically, thermally or mechanically bonded without being woven. In this connection, when fibers hold a certain shape by friction due to inter-fiber contacts or by entanglement thereof, the fibers are included in the category of mechanically bonded fibers. On the other hand, the terminology "woven fabric" used herein means a fabric in which warp and weft strands cross over, as generally defined.

With respect to the filter material of the present invention, the morphology and structure of any of the conventional filter materials can be employed depending on the situations and the desired characteristics (see, for example, PCT/JP91/01277 and PCT/JP92/01064).

With respect to the porous element in the present invention, the F value defined below is preferably in the range from 1.6 to 10 g/second/m, more preferably from 2.0 to 8 g/second/m, still more preferably from 2.4 to 6 g/second/m.

$$F \text{ value} = Q \times R \times 100$$

wherein Q represents the air permeability of porous element ($cm^3$/sec/$cm^2$) and R represents the weight of the porous element per $cm^2$ (g/$cm^2$), and wherein the air permeability is the volume ($cm^3$) of the air passing through the porous element per second per $cm^2$ of the porous element as measured at 20 °C at a pressure difference of 12.7 mmAq between opposite sides of the porous element in accordance with Japanese Industrial Standard (JIS) L-1096.

When the F value is smaller than 1.6, unfavorably, resistance to the passage of blood is increased, so that a good flow rate of blood is less likely to be obtained or a decrease in the flow rate of blood with time during filtration is likely to be high. On the other hand, when the F value is larger than 10, unfavorably, the frequency of contact between leukocytes and the porous element is lowered, so that leukocyte removal ratio is likely to become poor.

When the porous element is a fibrous fabric, representative examples of fiber materials usable for producing the fibrous fabric in the present invention include synthetic fibers, such as those of polyamide, an aromatic polyamide, polyester, polyacrylonitrile, polytrifluorochloroethylene, polymethyl methacrylate, polystyrene, polyethylene and polypropylene, regenerated fibers, such as those of cellulose acetate, cuprammonium rayon and viscose rayon, and natural fibers, such as those of flax, cotton, silk and animal hair. Of these examples, synthetic fibers are preferred because it is easy to obtain fibers having particular characteristics described below which are desired in the present invention. Among synthetic fibers, those of polyester, polyethylene and polypropylene are particularly preferred.

The fibrous fabric preferably has an average fiber diameter of from 0.3 to 5 μm, more preferably from 0.3 to 3.5 μm, still more preferably from 0.5 to 2.5 μm. When the average fiber diameter of the fibrous fabric is less than 0.3 μm, it is likely that a large pressure loss occurs when the filter material is used for treating a red cell product or a whole blood product and that red cells in the blood products being treated suffer from damage. On the other hand, when the average fiber diameter is larger than 5 μm, the frequency of contact between the fibers of the fibrous fabric and leukocytes becomes low, so that the effects of the present invention are unlikely to be fully exhibited.

In the present invention, the average fiber diameter of a fibrous fabric refers to a value determined by the following method. A portion of a fibrous fabric is sampled, and the resultant sample is photographed using a scanning electron microscope. In the sampling, an effective filtering area portion of the fibrous fabric is sectioned into 0.5 cm x 0.5 cm squares. From them, six squares are randomly sampled. In the random sampling, for example, an address is given to each of the above-mentioned square sections, and appropriate square sections are chosen, for example by a method in which the table of random numbers is used. With respect to each of the first sampled three square sections, the center portion of the upstream side surface is photographed at a magnification of 2500. On the other hand, with respect to each of the remaining three square sections, the center portion of the downstream side surface is photographed at the same magnification. In the photographing, a plurality of photographs are taken at a center portion and its neighborhood of each sampled square section. Photographing is continued until the number of fibers appearing in the photographs exceeds 100. The diameter is measured of each of the fibers appearing in the photographs. The term "diameter" used herein means the width of a fiber as viewed in a direction perpendicular to the fiber axis. The average fiber diameter is the quotient of the sum of all measured fiber diameters divided by the number of fibers, provided that when a plurality of fibers overlap each other to thereby cause the measurement of the fiber width to be infeasible due to the shadowing of other fibers, when a plurality of fibers are formed into a thick fiber through melt adhesion or the like, or when fibers of markedly different diameters are mixed, measurement data are omitted. Further, when the average fiber diameters are statistically significantly different between the upstream side and the downstream side of a fibrous fabric, it is not regarded as a single porous element. In this case, the upstream side and the downstream side are regarded as constituting different porous elements. A boundary is determined between the upstream side and the downstream side, and subsequently the average fiber diameters are separately measured for both of them.

When the porous element of the filter material of the present invention is a non-fibrous porous structure, such as a spongy structure, representative examples of materials for the non-fibrous porous structure include polyacrylonitrile, polysulfone, cellulose, cellulose acetate, polyvinyl acetal, polyester and poly(meth)acrylate.

With respect to the porous element in the present invention, the wetting time with pure water defined below is preferably less than 40 seconds, more preferably less than 30 seconds, still more preferably less than 10 seconds. When the wetting time with pure water is more than 40 seconds, wetting properties of the porous element with blood plasma (which has substantially the same surface tension as pure water) becomes poor, so that in an actual filtration operation, a smooth flowing of blood through the filter material is less likely to be attained.

The wetting time with pure water is measured by the following method. 10 $\mu$l of distilled water is dropped on the surface of a porous element and allowed to stand until the water drop is absorbed into the porous element. The time between the dropping and completion of the absorption of the drop by the porous element is taken. This operation is conducted 5 times and the average value of the 5 measurements is taken as the wetting time with pure water of the porous element.

In another aspect of the present invention, there is provided a filter apparatus for removing leukocytes from a whole blood product or a red cell product, which comprises a casing having a blood inlet and a blood outlet, and a filter material accommodated in the casing between the blood inlet and the blood outlet,

the filter material comprising at least one layer of a porous element having a three-dimensional network of continuous open pores, and wherein the porous element has, in a surface portion thereof, basic functional groups at a density of from $5 \times 10^{-5}$ to 0.1 meq/m$^2$ and nonionic hydrophilic groups, wherein a molar ratio of the basic functional groups to the nonionic hydrophilic groups is in the range from 0.6 to 6.

With respect to the filter apparatus of the present invention, the P value defined below is in the range of from 3 to 50 mmAq/g, preferably from 7 to 40 mmAq/g, more preferably from 13 to 30 mmAq/g.

$$\text{The P value} = \frac{\text{pressure loss of the air passing through the filter apparatus (mmAq)}}{\text{weight of the effective filtration area of the filter material (g)}}$$

The above-mentioned pressure loss of the air passing through the filter apparatus is the pressure difference as measured between the blood inlet and the blood outlet under conditions such that dry air is passed through the filter apparatus from the blood inlet of the apparatus to the blood outlet of the apparatus at a constant flow rate of 3 liters/minute at room temperature.

When the P value is smaller than 3, the leukocyte removal ratio tends to be poor. On the other hand, when the P value is larger than 50, the filtration time tends to be disadvantageously prolonged.

In the filter apparatus of the present invention, the filter material comprises one or more porous elements, each selected from the group consisting of a fibrous fabric and a non-fibrous porous structure which are mentioned above. When the filter material comprises a plurality of porous element layers, the porous element layers can also be a combination of a fibrous fabric layer and a non-fibrous porous structure layer.

The porous element preferably has an F value of from 1.6 to 10 g/second/m as defined above.

When a fibrous fabric as a porous element is packed into a casing, the packing density of the fibrous fabric is preferably in the range of from 0.08 to 0.4 g/cm$^3$, more preferably from 0.1 to 0.35 g/cm$^3$, still more preferably from 0.15 to 0.3 g/cm$^3$.

When the packing density is lower than 0.08 g/cm$^3$, unfavorably, the inter-fiber distances become too large, so that the frequency of contact between the fibers and leukocytes becomes low, thereby making it likely that the effects of the present invention cannot be fully exhibited. On the other hand, when the packing density is larger than 0.4 g/cm$^3$, unfavorably, the inter-fiber distances become too small, so that a pressure loss at the time of the passage of red cells between the fibers becomes impractically large. In the present invention, the terminology "packing density" of a fibrous fabric means a quotient of the weight of the effective filtration area of the fibrous fabric accommodated in a casing divided by the product of the effective filtration area multiplied by the thickness of the fibrous fabric.

When the filter material is packed in a casing so as to obtain a filter apparatus, the thickness of the filter material accommodated in the filter apparatus is preferably 1 to 10 mm, more preferably 2 to 8 mm, still more preferably 3 to 6 mm. When the thickness is less than 1 mm, the leukocyte removal ratio tends to become poor. On the other hand, when the thickness is more than 10 mm, the filtration time tends to be disadvantageously prolonged.

In the filter apparatus of the present invention, when the porous element is a fibrous non-woven fabric, it is preferred that the non-woven fabric comprise fibers having an average fiber diameter of from 0.3 to 5 $\mu$m.

The filter apparatus of the present invention may further comprise a customary prefilter material disposed upstream of the filter material of the present invention with respect to a flow direction in which a whole blood product or a red cell product to be treated for removal of leukocytes therefrom is adapted to be flowed. It is preferred that the prefilter material comprise a fibrous fabric comprising fibers having an average pore diameter which is larger than that of a porous element present at an upstream end portion of the filter material of the present invention, or comprise a non-fibrous porous structure having an average pore diameter which is larger than the average pore diameter of a porous element present at an upstream end portion of the filter material of the present invention.

In a more preferred form of the filter apparatus of the present invention, the filter material comprises a plurality of porous element layers having different average pore diameters, wherein the filter material has a gradient with respect to the average pore diameter such that the average pore diameter is substantially continuously or stepwise decreased from an upstream end portion to a downstream end portion of the filter material in a flow direction in which a whole blood product or a red cell product is adapted to be flowed and wherein a porous element layer which is present upstream of the downstream end portion of the filter material in the flow direction has an average pore diameter which is at least 1.2 times, more preferably at least 1.5 times that of a porous element layer present at the downstream end portion of a filter material.

In a filter material having the above-mentioned gradient with respect to the average pore diameter, the porous element layer present at the downstream end portion of the filter material (which has the smallest average pore diameter), has the largest ability to remove leukocytes, but the porous element layer present at the downstream end portion is most likely to suffer clogging with blood cells, such as leukocytes, due to the smallest average pore diameter. In the above more preferred form of the filter apparatus, a porous element layer which is present upstream of the downstream end portion of the filter material and has an average pore diameter which is at least 1.2 times, more preferably at least 1.5 times that of the porous element layer present at the downstream end portion of the filter material serves to roughly remove the leukocytes before blood material reaches the porous element layer present at the downstream end portion, so that the danger of occurrence of clogging of the porous element layer present at the downstream end portion is avoided.

With respect to the pore characteristics of the porous element, including an average pore diameter referred to above, a total pore volume and respective pore volumes of pores having specific pore diameters, are measured by mercury porosimetry (using Poresizer$^®$ 9320, PC 2A system, sold by Shimadzu Corporation, Japan). The terminology "average pore diameter" used herein means a diameter falling on a point where the amount of mercury penetrated into pores under a pressure is 50 %, provided that the amount of mercury penetrated into pores under the pressure is regarded as 0 % when no trace of mercury is penetrated into the pores of a porous element, and that the amount of mercury penetrated into pores under the pressure is regarded as 100 % when all the pores of the porous element are filled with mercury.

With respect to the measurement by mercury porosimetry under pressure using a mercury porosimeter, the pressure is generally in the range of from 0.1 to 180 psia.

In the measurement of each of the average pore diameter, the total pore volume and the respective pore volumes of pores having specific pore diameters, three or more samples are randomly taken from the porous element and measured, and an average of the values obtained with respect to the three or more samples is taken.

In the present invention, the porous element has an average pore diameter of preferably from 3 to 25 $\mu$m, more preferably from 3 to 20 $\mu$m, and wherein the sum of respective pore volumes of pores having a pore diameter of from 2 to 30 $\mu$m is preferably 85 % or more, more preferably 94 % or more, based on the total pore volume, the sum of respective pore volumes of pores having a pore diameter of more than 30 $\mu$m is preferably 6 % or less, more preferably 2 % or less, based on the total pore volume, and the sum of respective pore volumes of pores having a pore diameter of less than 2 $\mu$m is preferably less than 8 % or less, more preferably 5 % or less, based on the total pore volume.

In the filter apparatus of the present invention, the filter material preferably has an S/T value of from 50 to 300 cm, more preferably from 70 to 250 cm, still more preferably from 90 to 200 cm, in terms of the quotient of the effective filtration area (S) (cm$^2$) divided by the thickness (T) (cm) thereof. When the above-mentioned value of the quotient of the effective filtration area (cm$^2$) divided by the thickness (cm) thereof, is smaller than 50 cm, the flow rate of blood and the leukocyte removal ratio are likely to be largely lowered with time during filtration. On the other hand, when this value is larger than 300 cm, the thickness of the porous element is too small, so that when a red cell recovery as high as 90 % or more is desired (and hence the packing density of the filter material should be held down to a minimum), it becomes difficult to pack in the casing a filter material in an amount sufficient to attain the ability to reduce the amount of leukocytes in the blood product treated with the filter material to a level as low as $10^{-4}$ to $10^{-6}$ or less in terms of the leukocyte residual ratio. Herein, the term "thickness" means the distance between the inlet-side surface and outlet-side surface of the filter material (comprised of at least one layer of a porous element) accommodated in the filter apparatus.

With respect to the filter apparatus of the present invention, it is preferred that the whole blood product or red cell product be treated in an amount of 0.1 to 0.5 unit per g of the filter material. More preferably, the amount of a whole blood product or red cell product to be treated is 0.15 to 0.45 unit per g of the filter material. When the amount of a whole blood product or red cell product to be treated is smaller than 0.1 unit per g of the filter material, the ratio of waste blood product to be discarded together with a used filter material or a used filter apparatus is disadvantageously large, so that a red cell recovery is low. On the other hand, when the amount of a whole blood product or red cell product to be treated is larger than 0.5 unit per g of the filter material, the leukocyte removal ratio and the flow rate of a blood product are likely to be lowered.

In the present invention, one unit of a whole blood product is defined as 500 ml of collected whole blood having, added thereto, an anticoagulant, such as CPD solution, CPDA solution and ACD solution. One unit of a red cell product is defined as a red cell product prepared by removing a platelet-poor plasma, a platelet-rich plasma or buffy coat from one unit of a whole blood product by, for example, a centrifugal method, or a red cell product prepared by adding to the above-mentioned red cell product a red cell preservative, such as SAGM, AS-1, AS-3 or MAP.

In the filter apparatus of the present invention, it is preferred that the basic functional groups be tertiary amino groups.

In the filter apparatus of the present invention, it is preferred that the nonionic hydrophilic groups be selected from the group consisting of hydroxyl groups, polyethylene oxide groups and a mixture thereof.

In still another aspect of the present invention, there is provided a method for removing leukocytes from a whole blood product or a red cell product, comprising subjecting a whole blood product or a red cell product to filtration with a filter apparatus,

the filter apparatus comprising a casing having a blood inlet and a blood outlet, and a filter material accommodated in the casing between the blood inlet and the blood outlet,

the filter material comprising at least one layer of a porous element having a three-dimensional network of continuous open pores, and wherein the porous element has, in a surface portion thereof, basic functional groups at a density of from $5 \times 10^{-5}$ to 0.1 meq/m$^2$ and nonionic hydrophilic groups, wherein a molar ratio of the basic functional groups to the nonionic hydrophilic groups is in the range from 0.6 to 6,

the filter apparatus having a P value of from 3 to 50 mmAq/g, the P value being defined above,

the whole blood product or red cell product being treated in an amount of 0.1 to 0.5 unit per g of the filter material.

In a more preferred form of the method of the present invention, the filter material accommodated in the filter apparatus comprises a plurality of porous element layers having different average pore diameters, wherein the filter material has a gradient with respect to the average pore diameter such that the average pore diameter is substantially continuously or stepwise decreased from an upstream end portion to a downstream end portion of the filter material in a flow direction in which a whole blood product or a red cell product is adapted to be flowed and wherein a porous element layer which is present upstream of the downstream end portion of the filter material in the flow direction has an average pore diameter which is at least 1.2 times, more preferably at least 1.5 times that of a porous element layer present at the downstream end portion of the filter material.

In the method of the present invention, the filter material preferably has an S/T value of from 50 to 300 cm, more preferably from 70 to 250 cm, still more preferably from 90 to 200 cm, in terms of the quotient of the effective filtration area (S) (cm$^2$) divided by the thickness (T) (cm) thereof.

In the method of the present invention, it is also preferred that the basic functional groups be tertiary amino groups and the nonionic hydrophilic groups be selected from the group consisting of hydroxyl groups, polyethylene oxide groups and a mixture thereof.

With respect to other factors of the filter apparatus and filter material used in the method of the present invention, reference can be made to the above descriptions regarding the filter apparatus and the filter material.

With the filter material, filter apparatus and method of the present invention, it has become possible that the amount of leukocytes in a whole blood product or red cell product can be reduced to a level as low as $10^{-4}$ to $10^{-6}$ or less in terms of the leukocyte residual ratio while exhibiting not only a red cell recovery as high as 90 % or more but also a high filtration rate.

Preferred Embodiment of The Invention

The present invention will now be described in greater detail with reference to the following Examples and Comparative Examples, which should not be construed as limiting the scope of the present invention.

In the following Examples and Comparative Examples, a fibrous fabric which is not coated with a copolymer and a fibrous fabric which is coated with a copolymer are, respectively, referred to as "fibrous fabric" and "filter material".

The average fiber diameter, packing density and thickness of coated fibrous fabrics (filter materials) described in the following Examples and Comparative Examples are substantially identical to those of non-coated fibrous fabrics corresponding to the coated fibrous fabrics.

Example 1

Dimethylaminoethyl methacrylate (hereinafter referred to as "DM") and 2-hydroxyethyl methacrylate (hereinafter referred to as "HEMA") are added to special grade ethanol [Wako special grade ethanol (sold by Woko Pure Chemical Industries, Ltd., Japan)] so that the final DM and HEMA concentrations of the resultant mixture become 0.4 mole/liter and 0.6 mole/liter, respectively, and the volume of the resultant mixture becomes 250 ml. To the obtained mixture is added V-65 [tradename of 2,2'-azobis(2,4-dimethyl-valeronitrile) manufactured and sold by Wako Pure Chemical Industries, Ltd., Japan] as a polymerization initiator in a concentration of 0.01 mole/liter and then, polymerization is carried out at 45 °C for 4.5 hours in nitrogen atmosphere. The resultant reaction mixture is dropwise added to distilled water to produce deposits in the surface portion of the water. The collected and subjected to lyophilization to thereby obtain a copolymer of DM and HEMA.

As a first fibrous fabric, a polyester melt-blown non-woven fabric having an average fiber diameter of 1.8 $\mu$m is packed in a casing having a blood inlet and a blood outlet and having an effective filtration area of 67 mm x 67 mm so that the packing density and thickness of the first fibrous fabric become 0.18 g/cm$^3$ and 1.5 mm, respectively. Then, as a second fibrous fabric, another polyester melt-blown non-woven fabric having an average fiber diameter of 1.2 $\mu$m is packed downstream of the first fibrous fabric so that the packing density and thickness of the second fibrous fabric become 0.2 g/cm$^3$ and 3 mm, respectively. Then, a 0.01 g/dl solution of the above-obtained copolymer in guaranteed ethanol is charged in the above-mentioned casing and allowed to stand still for one minute. Subsequently, the solution is expelled from the casing by flowing nitrogen gas to the casing. Nitrogen gas is further flowed at a flow rate of 5 liters/min for 10 minutes and then, the resultant copolymer-coated fibrous fabrics are subjected to vacuum-drying at 70 °C for 7 hours, to thereby obtain first and second filter materials accommodated in the casing. Thus, a filter apparatus for removing leukocytes, which is comprised of the casing and the first and second filter materials packed therein, is prepared.

The molar ratio of the basic functional groups to the nonionic hydrophilic groups present in the surface of the filter material is 0.67, and the density of the basic functional groups present in the surface of the filter material is 9.5 x 10$^{-4}$ meq/m$^2$. The F values of the first filter material (having an average fiber diameter of 1.8 $\mu$m) and the second filter material (having an average fiber diameter of 1.2 $\mu$m) are, respectively, 5.6 g/sec/m and 3.3 g/sec/m. With respect to each of the first and second filter materials, the wetting time with pure water is 16 seconds.

The P value of the filter apparatus is 13.8 mmAq/g.

456 ml of a whole blood product prepared by adding 56 ml of CPD solution to 400 ml of whole blood is subjected to centrifugation within 8 hours after collection of the whole blood to separate 200 ml of platelet-rich plasma from the whole blood product, thereby obtaining a red cell concentrate. The red cell concentrate is placed in a blood bag and stored at 4 °C for 3 days (hematocrit: 69 %). 320 ml of the resultant red cell concentrate is then allowed to stand at room temperature (26 °C) until the temperature of the concentrate reaches 25 °C. Then, the red cell concentrate is subjected to filtration by the above-mentioned filter apparatus. That is, the blood bag containing the red cell concentrate is connected to the blood inlet of the filter apparatus through a blood tubing and then, a pressure of 100 mmHg is applied to the blood bag by a pressure cuff, thereby forcibly filling the filter apparatus with the red cell concentrate. After filling the filter apparatus with the red cell concentrate, the filtration is performed with a head of 1.0 m to collect a filtered red cell concentrate in a collection bag which has been connected to the blood outlet of the filter apparatus through a blood tubing. At the time when no blood remains in the blood bag any longer, the filtration is completed, and the collection bag is cut off from the filter apparatus by cutting the blood tubing at an intermediate position thereof 20 to 30 cm downstream of the blood outlet of the filter apparatus, thereby recovering a leukocyte-removed red cell concentrate (hereinafter frequently referred to as "recovered liquid") in the collection bag.

The red cell concentrate before filtration (hereinafter referred to as "pre-filtration liquid") and the recovered liquid are measured with respect to the volume, the hematocrit and the number of leukocytes, thereby determining the red cell recovery and the leukocyte residual ratio.

Red Cell Recovery = [Recovered Liquid Volume x Hematocrit
(Recovered Liquid)]/[Pre-filtration Liquid Volume x Hematocrit (Pre-filtration Liquid)].

Leukocyte Residual Ratio = [Number of Leukocytes (Recovered Liquid)]/[Pre-filtration Liquid Volume x
Leukocyte Concentration (Pre-filtration Liquid)].

With respect to the volumes of the pre-filtration liquid and the recovered liquid, values obtained by dividing the weights of these liquids by 1.075 (a representative value of the specific gravity of a red cell concentrate) are taken as the respective volumes. Further, the measurement of the leukocyte concentration of the pre-filtration liquid is performed by the following method.

The measurement of the leukocyte concentration of the pre-filtration liquid: A pre-filtration liquid diluted 10-fold with Türk's reagent is injected into a Burker-Türk type hemocytometer (manufactured and sold by Erma, Japan) and the total number of leukocytes present in four squares (0.4 $\mu$l in volume) is counted by means of an optical microscope and the obtained total number was taken as $n_1$.

$$\text{Leukocyte Concentration} = n_1 \times (1/4) \times 10^5 \text{cells/ml}.$$

The measurement of the number of leukocytes contained in a recovered liquid is performed by the method described below.

100 ml of an EBSS solution containing 5 % FICOLL 400 DL and 1 % bovine serum albumin (BSA) (both of which FICOLL and BSA are manufactured and sold by Sigma Chemical Co., St. Louis, the U.S.A) (hereinafter the EBSS solution containing FICOLL and BSA is referred to as "FICOLL solution") is introduced into a bag containing 100 ml of the recovered liquid while shaking to facilitate mixing. Then, the bag is placed in a plasma expressor and allowed to stand still for 40 minutes. After that period, a supernatant is gently collected without disturbing a precipitated layer of red cells. Then, 100 ml of a FICOLL solution is again introduced into the bag, and the same procedure as described above is repeated. The supernatant collected by the collecting operation thus conducted twice is transferred to a centrifuge tube being CORNING 25350-250 (manufactured and sold by Corning Laboratory Science Company, New York, the U.S.A.) and centrifuged at 840 x g for 15 minutes to deposit pellets. Subsequently, the supernatant is discarded by means of an aspirator so carefully as not to withdraw the pellets. 200 ml of a hemolysis solution (a 1.145 % ammonium oxalate solution in Sorenson's buffer, pH 6.8) is introduced into a tube and the tube is shaken to facilitate mixing, immediately followed by centrifugation at 468 x g for 10 minutes. Subsequently, the supernatant is discarded by means of an aspirator while taking the same care as described above.

The pellets in the tube are collected into a 15 ml centrifuge tube and the same hemolysis solution as mentioned above is added thereto so that the total volume becomes 15 ml. The tube is allowed to stand still at room temperature for 10 minutes and then centrifuged at 468 x g for 10 minutes. Part of the supernatant is carefully discarded so that the volume of the contents including the pellets becomes 0.5 ml. The liquid in the tube containing the precipitates is stirred well to obtain a single cell suspension, and 50 $\mu$l of a fluorescent dyeing solution [69.9 mg/l Acridine Orange (manufactured and sold by Nacalai Tesque Inc., Japan) in an EBSS solution containing 1 % BSA] is added, followed by stirring. The resultant liquid is injected into six hemocytometers of improved Neubauer type (manufactured and sold by Reichert Co., Buffalo, the U.S.A.) and the total number of leukocytes present in 108 squares (10.8 $\mu$l in volume) are counted by means of an epi-fluorescence microscope (manufactured and sold by Nicon Corp., Japan).

From the total number ($n_2$) of leukocytes, the number of leukocytes (of recovered liquid) is calculated.

$$\text{Number of Leukocytes (Recovered Liquid)} = \underline{n_2 \times (1/108) \times 10^4} \times 0.55 \times (1/0.55) \times [\text{Recovered Liquid Volume (ml)}/100].$$

The underlined portion in the formula represents the leukocyte concentration (cells/ml) in a concentrated liquid (hereinafter referred to as "concentrate") obtained by concentrating 100 ml of the recovered liquid using a FICOLL solution to a volume of 0.55 ml. The leukocyte concentration is multiplied by the volume of the concentrate (0.55 ml) to obtain the number of leukocytes. The reason why the thus obtained number of leukocytes is further divided by 0.55 is that the recovery of leukocytes attained by means of a FICOLL solution is 55 %.

As a result of the above calculations, it is found that the leukocyte residual ratio is $10^{-4.8}$ and the red cell recovery is 91.9 %.

The time required for conducting filtration of the red cell concentrate is less than 25 minutes, and the flow rate of the red cell concentrate is good.

Comparative Example 1

A filter apparatus is prepared in substantially the same manner as in Example 1 except that the coating of the fibrous fabrics with a copolymer is not conducted.

Neither basic functional groups nor nonionic hydrophilic groups are detected in the surfaces of the first and second fibrous fabrics, so that the molar ratio of the basic functional groups to the nonionic hydrophilic groups present in the surface of the fibrous fabrics cannot be measured.

The F values of the first fibrous fabric (having an average fiber diameter of 1.8 $\mu$m) and the second fibrous fabric (having an average fiber diameter of 1.2 $\mu$m) are 5.7 g/sec/m and 3.2 g/sec/m, respectively. With respect to each of the first and second fibrous fabrics, the wetting time with pure water is more than 1 minute.

The P value of the filter apparatus is 14.7 mmAq/g.

Using the filter apparatus, substantially the same filtration operation as in Example 1 is performed.

As a result, the leukocyte residual ratio is $10^{-3.7}$ and the red cell recovery is 90.6 %.

Example 2

A filter apparatus is prepared in substantially the same manner as in Example 1 except that the concentrations of DM and HEMA in the ethanol solution are changed to 0.5 mole/liter and 0.5 mole/liter, respectively.

The molar ratio of the basic functional groups to the nonionic hydrophilic groups present in the surface of the filter material is 0.97, and the density of the basic functional groups present in the surface of the filter material is 1.2 x $10^{-3}$ meq/m$^2$. The F values of the first filter material (having an average fiber diameter of 1.8 $\mu$m) and the second filter material (having an average fiber diameter of 1.2 $\mu$m) are 5.5 g/sec/m and 3.3 g/sec/m, respectively. With respect to each of the first and second filter materials, the wetting time with pure water is 19 seconds.

The P value of the filter apparatus is 14.2 mmAq/g.

Using the filter apparatus, a filtration operation is conducted under substantially the same conditions as in Example 1.

As a result, the leukocyte residual ratio is $10^{-5}$ and the red cell recovery is 92.7 %.

The time required for conducting filtration of the red cell concentrate is less than 25 minutes, and the flow rate of the red cell concentrate is good.

Example 3

A filter apparatus is prepared in substantially the same manner as in Example 1 except that the concentrations of DM and HEMA in the ethanol solution are changed to 0.6 mole/liter and 0.4 mole/liter, respectively.

The molar ratio of the basic functional groups to the nonionic hydrophilic groups present in the surface of the filter material is 1.49, and the density of the basic functional groups present in the surface of the filter material is 1.4 x $10^{-3}$ meq/m$^2$. The F values of the first filter material (having an average fiber diameter of 1.8 $\mu$m) and the second filter material (having an average fiber diameter of 1.2 $\mu$m) are 5.4 g/sec/m and 3.0 g/sec/m, respectively. With respect to each of the first and second filter materials, the wetting time with pure water is 20 seconds.

The P value of the filter apparatus is 15.1 mmAq/g.

Using the filter apparatus, a filtration operation is conducted under substantially the same conditions as in Example 1.

As a result, the leukocyte residual ratio is $10^{-5.2}$ and the red cell recovery is 92.0 %.

The time required for conducting filtration of the red cell concentrate is less than 25 minutes, and the flow rate of the red cell concentrate is good.

Example 4

A filter apparatus is prepared in substantially the same manner as in Example 1 except that the concentrations of DM and HEMA in the ethanol solution are changed to 0.7 mole/liter and 0.3 mole/liter, respectively.

The molar ratio of the basic functional groups to the nonionic hydrophilic groups present in the surface of the filter material is 2.31, and the density of the basic functional groups present in the surface of the filter material is 1.6 x $10^{-3}$ meq/m$^2$. The F values of the first filter material (having an average fiber diameter of 1.8 $\mu$m) and the second filter material (having an average fiber diameter of 1.2 $\mu$m) are 5.4 g/sec/m and 3.1 g/sec/m, respectively. With respect to each of the first and second filter materials, the wetting time with pure water is 22 seconds.

The P value of the filter apparatus is 14.3 mmAq/g.

Using the filter apparatus, a filtration operation is conducted under substantially the same conditions as in Example 1.

As a result, the leukocyte residual ratio is $10^{-5.2}$ and the red cell recovery is 91.8 %.

The time required for conducting filtration of the red cell concentrate is less than 25 minutes, and the flow rate of the red cell concentrate is good.

## Example 5

A filter apparatus is prepared in substantially the same manner as in Example 1 except that the concentrations of DM and HEMA in the ethanol solution are changed to 0.8 mole/liter and 0.2 mole/liter, respectively.

The molar ratio of the basic functional groups to the nonionic hydrophilic groups present in the surface of the filter material is 3.98, and the density of the basic functional groups present in the surface of the filter material is $1.8 \times 10^{-3}$ meq/m$^2$. The F values of the first filter material (having an average fiber diameter of 1.8 $\mu$m) and the second filter material (having an average fiber diameter of 1.2 $\mu$m) are 5.6 g/sec/m and 3.5 g/sec/m, respectively. With respect to each of the first and second filter materials, the wetting time with pure water is 22 seconds.

The P value of the filter apparatus is 13.9 mmAq/g.

Using the filter apparatus, a filtration operation is conducted under substantially the same conditions as in Example 1.

As a result, the leukocyte residual ratio is $10^{-5.0}$ and the red cell recovery is 90.5 %.

The time required for conducting filtration of the red cell concentrate is less than 25 minutes, and the flow rate of the red cell concentrate is good.

## Comparative Example 2

A filter apparatus is prepared in substantially the same manner as in Example 1 except that diethylaminoethyl methacrylate (hereinafter referred to simply as "DE") is used instead of DM, and the concentrations of DE and HEMA in the ethanol solution are changed to 0.2 mole/liter and 0.8 mole/liter, respectively.

The molar ratio of the basic functional groups to the nonionic hydrophilic groups present in the surface of the filter material is 0.25, and the density of the basic functional groups present in the surface of the filter material is $4.7 \times 10^{-4}$ meq/m$^2$. The F values of the first filter material (having an average fiber diameter of 1.8 $\mu$m) and the second filter material (having an average fiber diameter of 1.2 $\mu$m) are 5.3 g/sec/m and 3.3 g/sec/m, respectively. With respect to each of the first and second filter materials, the wetting time with pure water is 11 seconds.

The P value of the filter apparatus is 14.9 mmAq/g.

Using the filter apparatus, a filtration operation is conducted under substantially the same conditions as in Example 1.

As a result, the leukocyte residual ratio is $10^{-3.8}$ and the red cell recovery is 93.0 %.

## Comparative Example 3

A filter apparatus is prepared in substantially the same manner as in Example 1 except that the concentrations of DM and HEMA in the ethanol solution are changed to 0.3 mole/liter and 0.7 mole/liter, respectively.

The molar ratio of the basic functional groups to the nonionic hydrophilic groups present in the surface of the filter material is 0.43, and the density of the basic functional groups present in the surface of the filter material is $7.2 \times 10^{-4}$ meq/m$^2$. The F values of the first filter material (having an average fiber diameter of 1.8 $\mu$m) and the second filter material (having an average fiber diameter of 1.2 $\mu$m) are 5.6 g/sec/m and 3.6 g/sec/m, respectively. With respect to each of the first and second filter materials, the wetting time with pure water is 13 seconds.

The P value of the filter apparatus is 15.3 mmAq/g.

Using the filter apparatus, a filtration operation is conducted under substantially the same conditions as in Example 1.

As a result, the leukocyte residual ratio is $10^{-3.9}$ and the red cell recovery is 92.3 %.

## Comparative Example 4

A filter apparatus is prepared in substantially the same manner as in Example 1 except that the concentrations of DM and HEMA in the ethanol solution are changed to 0.9 mole/liter and 0.1 mole/liter, respectively.

The molar ratio of the basic functional groups to the nonionic hydrophilic groups present in the surface of the filter material is 8.94, and the density of the basic functional groups present in the surface of the filter material is $2.0 \times 10^{-3}$ meq/m$^2$. The F values of the first filter material (having an average fiber diameter of 1.8 $\mu$m) and the second filter material (having an average fiber diameter of 1.2 $\mu$m) are 5.8 g/sec/m and 3.4 g/sec/m, respectively. With respect to each of the first and second filter materials, the wetting time with pure water is 38 seconds.

The P value of the filter apparatus is 14.2 mmAq/g.

Using the filter apparatus, a filtration operation is conducted under substantially the same conditions as in Example 1.

As a result, the leukocyte residual ratio is $10^{-3.6}$ and the red cell recovery is 91.1 %.

Comparative Example 5

A filter apparatus is prepared in substantially the same manner as in Example 1 except that only DE having basic functional groups is dissolved in the same Wako special grade ethanol as mentioned above so that the final concentration becomes 1 mole/liter.

Nonionic hydrophilic groups are not detected in the surface of the filter material. The density of the basic functional groups present in the surface of the filter material is $1.8 \times 10^{-3}$ meq/m$^2$. The F values of the first filter material (having an average fiber diameter of 1.8 $\mu$m) and the second filter material (having an average fiber diameter of 1.2 $\mu$m) are 5.5 g/sec/m and 3.4 g/sec/m, respectively. With respect to each of the first and second filter materials, the wetting time with pure water is 45 seconds.

The P value of the filter apparatus is 14.7 mmAq/g.

Using the filter apparatus, a filtration operation is conducted under substantially the same conditions as in Example 1.

As a result, the leukocyte residual ratio is $10^{-3.8}$ and the red cell recovery is 89.7 %.

Comparative Example 6

A filter apparatus is prepared in substantially the same manner as in Example 2 except that the concentration of the copolymer in the ethanol solution is changed to 0.0003 g/dl.

The molar ratio of the basic functional groups to the nonionic hydrophilic groups present in the surface of the filter material is 0.97, and the density of the basic functional groups present in the surface of the filter material is $3.5 \times 10^{-5}$ meq/m$^2$. The F values of the first filter material (having an average diameter of 1.8 $\mu$m) and the second filter material (having an average diameter of 1.2 $\mu$m) are 6.0 g/sec/m and 3.5 g/sec/m, respectively. With respect to each of the first and second filter materials, the wetting time with pure water is 19 seconds.

The P value of the filter apparatus is 14.5 mmAq/g.

Using the filter apparatus, a filtration operation is conducted under substantially the same conditions as in Example 2.

As a result, the leukocyte residual ratio is $10^{-3.8}$ and the red cell recovery is 90.9 %.

Comparative Example 7

A filter apparatus is prepared in substantially the same manner as in Example 2 except that the concentration of the copolymer in the ethanol solution is changed to 1 g/dl.

The molar ratio of the basic functional groups to the nonionic hydrophilic groups present in the surface of the filter material is 0.97, and the density of the basic functional groups present in the surface of the filter material is $1.2 \times 10^{-1}$ meq/m$^2$. The F values of the first filter material (having an average diameter of 1.8 $\mu$m) and the second filter material (having an average diameter of 1.2 $\mu$m) are 2.5 g/sec/m and 1.3 g/sec/m, respectively. With respect to each of the first and second filter materials, the wetting time with pure water is 23 seconds.

The P value of the filter apparatus is 53.0 mmAq/g.

Using the filter apparatus, a filtration operation is conducted under substantially the same conditions as in Example 2.

When 320 ml of a red cell concentrate is subjected to filtration by the resultant filter apparatus, the flow rate of the red cell concentrate is lowered so drastically that only 60 ml of the red cell concentrate is recovered (the red cell recovery: 16.3 %), so that the determination of the leukocyte residual ratio cannot be conducted. Furthermore, in the leukocyte-removed red cell concentrate, it is observed that hemolysis of red cells occurs markedly.

The results of the above Examples 1 to 5 and Comparative Examples 1 to 7 are shown in Table 1.

Table 1

| | Molar ratio of basic functional groups to nonionic hydrophilic groups | Surface basic functional group density (meq/m$^2$) | Leukocyte residual ratio | Red cell recovery (%) |
|---|---|---|---|---|
| Example 1 | 0.67 | 9.5 x 10$^{-4}$ | 10$^{-4.8}$ | 91.9 |
| Example 2 | 0.97 | 1.2 x 10$^{-3}$ | 10$^{-5.0}$ | 92.7 |
| Example 3 | 1.49 | 1.4 x 10$^{-3}$ | 10$^{-5.2}$ | 92.0 |
| Example 4 | 2.31 | 1.6 x 10$^{-3}$ | 10$^{-5.2}$ | 91.8 |
| Example 5 | 3.98 | 1.8 x 10$^{-3}$ | 10$^{-5.0}$ | 90.5 |
| Comparative Example 1 | - | - | 10$^{-3.7}$ | 90.6 |
| Comparative Example 2 | 0.25 | 4.7 x 10$^{-4}$ | 10$^{-3.8}$ | 93.0 |
| Comparative Example 3 | 0.43 | 7.2 x 10$^{-4}$ | 10$^{-3.9}$ | 92.3 |
| Comparative Example 4 | 8.94 | 2.0 x 10$^{-3}$ | 10$^{-3.6}$ | 91.1 |
| Comparative Example 5 | - | 1.8 x 10$^{-3}$ | 10$^{-3.8}$ | 89.7 |
| Comparative Example 6 | 0.97 | 3.5 x 10$^{-5}$ | 10$^{-3.8}$ | 90.9 |
| Comparative Example 7 | 0.97 | 1.2 x 10$^{-1}$ | - | 16.3 |

Example 6

Dimethylaminoethyl acrylate (hereinafter referred to as "DMA") and 2-hydroxyethyl acrylate (hereinafter referred to as "HEA") are added to the same Wako special grade ethanol as mentioned above so that the final DMA and HEA concentrations of the resultant mixture become 0.4 mole/liter and 0.6 mole/liter, respectively, and the volume of the resultant mixture becomes 250 ml. To the obtained mixture is added V-65 as a polymerization initiator in a concentration of 0.01 mole/liter and then, polymerization is carried out at 45 °C for 4.5 hours in nitrogen atmosphere. The resultant reaction mixture is dropwise added to n-hexane to thereby obtain a precipitate. The obtained precipitate is collected and lyophilized to thereby obtain a copolymer of DMA and HEA.

As prefilter materials for removing gels and macro- and micro-aggregates, three types of spun bonded non-woven fabrics made of polyester fibers respectively having average fiber diameters of 32 μm, 19 μm and 13 μm are packed in this order relative to the direction of blood flowing in a casing having a blood inlet and a blood outlet and having an effective filtration area of 67 mm x 67 mm so that the average packing density and total thickness of the three fabrics become 0.28 g/cm$^3$ and 0.9 mm, respectively. Then, as prefilter materials for removing microaggregates, Panelon PF860 (trade name of a polyester non-woven fabric manufactured and sold by Dynic Co., Japan) having an average fiber diameter of 12 μm and a spun laced non-woven fabric having an average fiber diameter of 5.1 μm are packed in this order relative to the direction of the blood flowing at a position downstream of the above-mentioned prefilter materials for removing gels and macro- and micro-aggregates so that the average packing density and total thickness of the two fabrics become 0.20 g/cm$^3$ and 0.7 mm, respectively. Further, as a first fibrous fabric for removing leukocytes, a polyester melt-blown non-woven fabric having an average fiber diameter of 1.8 μm is packed downstream of the above-mentioned prefilter materials for removing micro-aggregates so that the packing density and thickness of the first fibrous fabric become 0.18 g/cm$^3$ and 1.5 mm, respectively. Then, as a second fibrous fabric for removing leukocytes, another polyester melt-blown non-woven fabric having an average fiber diameter of 1.2 μm is packed downstream of the above-mentioned first fibrous fabric so that the packing density and thickness of the second fibrous fabric become 0.2 g/cm$^3$

and 3 mm, respectively. Then, a 0.01 g/dl solution of the above-obtained copolymer in the same Wako special grade ethanol as mentioned above is charged in the above-mentioned casing and allowed to stand still for one minute. Subsequently, the solution is expelled from the casing by flowing nitrogen gas to the casing. Nitrogen gas is further flowed at a flow rate of 5 liters/min for 10 minutes and then, the resultant copolymer-coated fibrous fabrics are subjected to vacuum-drying at 70 °C for 7 hours, to thereby obtain the prefilter materials for removing gels and macro- and micro-aggregates, the prefilter materials for removing micro-aggregates and the first and second filter materials for removing leukocytes accommodated in the casing. Thus, a filter apparatus for removing leukocytes, which is composed of the casing and, packed therein, the prefilter materials for removing gels and macro- and micro-aggregates, the prefilter materials for removing microaggregates and the first and second filter materials for removing leukocytes, is prepared.

The molar ratio of the basic functional groups to the nonionic hydrophilic groups present in the surface of the filter material is 0.66, and the density of the basic functional groups present in the surface of the filter material is $1.1 \times 10^{-3}$ meq/m$^2$. The F values of the first filter material (having an average diameter of 1.8 μm) and the second filter material (having an average diameter of 1.2 μm) are 5.5 g/sec/m and 3.3 g/sec/m, respectively. With respect to each of the first and second filter materials, the wetting time with pure water is less than one second.

The P value of the filter apparatus is 14.3 mmAq/g.

456 ml of a whole blood product prepared by adding 56 ml of CPD solution to 400 ml of whole blood is subjected to centrifugation within 8 hours after collection of the whole blood to separate 200 ml of platelet-rich plasma from the whole blood product, thereby obtaining a red cell concentrate. The red cell concentrate is placed in a blood bag and stored at 4 °C for 14 days (hematocrit: 70 %). 320 ml of the resultant red cell concentrate is then allowed to stand at room temperature (26 °C) until the temperature of the concentrate reaches 25 °C. Then, the red cell concentrate is subjected to filtration by the above-mentioned filter apparatus. A filtration operation is conducted in substantially the same manner as in Example 1.

As a result, the leukocyte residual ratio is $10^{-4.3}$ and the red cell recovery is 90.5 %.

The time required for conducting filtration of the red cell concentrate is less than 25 minutes, and the flow rate of the red cell concentrate is good.

Example 7

A filter apparatus is prepared in substantially the same manner as in Example 6 except that the concentrations of DMA and HEA in the ethanol solution are changed to 0.7 mole/liter and 0.3 mole/liter, respectively.

The molar ratio of the basic functional groups to the nonionic hydrophilic groups present in the surface of the filter material is 2.3, and the density of the basic functional groups present in the surface of the filter material is $1.7 \times 10^{-3}$ meq/m$^2$. The F values of the first filter material (having an average fiber diameter of 1.8 μm) and the second filter material (having an average fiber diameter of 1.2 μm) are, respectively, 5.6 g/sec/m and 3.3 g/sec/m. With respect to each of the first and second filter materials, the wetting time with pure water is 8 seconds.

The P value of the filter apparatus is 14.7 mmAq/g.

Using the filter apparatus, a filtration operation is conducted under substantially the same conditions as in Example 6.

As a result, the leukocyte residual ratio is $10^{-4.3}$ and the red cell recovery is 90 %.

The time required for conducting filtration of the red cell concentrate is less than 25 minutes, and the flow rate of the red cell concentrate is good.

Example 8

A filter apparatus is prepared in substantially the same manner as in Example 6 except that the concentrations of DMA and HEA in the ethanol solution are changed to 0.85 mole/liter and 0.15 mole/liter, respectively.

The molar ratio of the basic functional groups to the nonionic hydrophilic groups present in the surface of the filter material is 5.7, and the density of the basic functional groups present in the surface of the filter material is $2.0 \times 10^{-3}$ meq/m$^2$. The F values of the first filter material (having an average fiber diameter of 1.8 μm) and the second filter material (having an average fiber diameter of 1.2 μm) are, respectively, 5.6 g/sec/m and 3.5 g/sec/m. With respect to each of the first and second filter materials, the wetting time with pure water is 15 seconds.

The P value of the filter apparatus is 15.0 mmAq/g.

Using the filter apparatus, a filtration operation is conducted under substantially the same conditions as in Example 6.

As a result, the leukocyte residual ratio is $10^{-4.0}$ and the red cell recovery is 90.1 %.

The time required for conducting filtration of the red cell concentrate is less than 25 minutes, and the flow rate of the red cell concentrate is good.

Comparative Example 8

A filter material is prepared in substantially the same manner as in Example 6 except that coating of the fibrous fabrics with the copolymer is not conducted.

Neither basic functional groups nor nonionic hydrophilic groups are detected in the surface of the first and second fibrous fabrics, so that the molar ratio of the basic functional groups to the nonionic hydrophilic groups present in the surface of the fibrous fabrics cannot be measured.

The F values of the first fibrous fabric (having an average fiber diameter of 1.8 $\mu$m) and the second fibrous fabric (having an average fiber diameter of 1.2 $\mu$m) are, respectively, 5.6 g/sec/m and 3.6 g/sec/m. With respect to each of the first and second fibrous fabrics, the wetting time with pure water is more than 1 minute.

The P value of the filter apparatus is 15.1 mmAq/g.

Using the filter apparatus, a filtration operation is conducted under substantially the same conditions as in Example 6.

As a result, the leukocyte residual ratio is $10^{-3.4}$ and the red cell recovery is 88.2 %.

Comparative Example 9

A filter apparatus is prepared in substantially the same manner as in Example 6 except that the concentrations of DMA and HEA in the ethanol solution are changed to 0.3 mole/liter and 0.7 mole/liter, respectively.

The molar ratio of the basic functional groups to the nonionic hydrophilic groups present in the surface of the filter material is 0.43, and the density of the basic functional groups present in the surface of the filter material is 8.0 x $10^{-4}$ meq/m$^2$. The F values of the first filter material (having an average fiber diameter of 1.8 $\mu$m) and the second filter material (having an average fiber diameter of 1.2 $\mu$m) are, respectively, 5.5 g/sec/m and 3.7 g/sec/m. With respect to each of the first and second filter materials, the wetting time with pure water is less than one second.

The P value of the filter apparatus is 13.9 mmAq/g.

Using the filter apparatus, a filtration operation is conducted under substantially the same conditions as in Example 6.

As a result, the leukocyte residual ratio is $10^{-3.6}$ and the red cell recovery is 90.4 %.

Comparative Example 10

A filter apparatus is prepared in substantially the same manner as in Example 6 except that the concentrations of DMA and HEA in the ethanol solution are changed to 0.9 mole/liter and 0.1 mole/liter, respectively.

The molar ratio of the basic functional groups to the nonionic hydrophilic groups present in the surface of the filter material is 7.7, and the density of the basic functional groups present in the surface of the filter material is 2.2 x $10^{-3}$ meq/m$^2$. The F values of the first filter material (having an average fiber diameter of 1.8 $\mu$m) and the second filter material (having an average fiber diameter of 1.2 $\mu$m) are, respectively, 5.7 g/sec/m and 3.5 g/sec/m. With respect to each of the first and second filter materials, the wetting time with pure water is 16 seconds.

The P value of the filter apparatus is 14.8 mmAq/g.

Using the filter apparatus, a filtration operation is conducted under substantially the same conditions as in Example 6.

As a result, the leukocyte residual ratio is $10^{-3.6}$ and the red cell recovery is 89.8 %.

Comparative Example 11

A filter apparatus is prepared in substantially the same manner as in Example 6 except that the concentration of the copolymer in the ethanol solution is changed to 0.0003 g/dl.

The molar ratio of the basic functional groups to the nonionic hydrophilic groups present in the surface of the filter material is 0.66, and the density of the basic functional groups present in the surface of the filter material is 3.2 x $10^{-5}$ meq/m$^2$. The F values of the first filter material (having an average fiber diameter of 1.8 $\mu$m) and the second filter material (having an average fiber diameter of 1.2 $\mu$m) are, respectively, 5.4 g/sec/m and 3.4 g/sec/m. With respect to each of the first and second filter materials, the wetting time with pure water is less than one second.

The P value of the filter apparatus is 13.8 mmAq/g.

Using the filter apparatus, a filtration operation is conducted under substantially the same conditions as in Example 6.

As a result, the leukocyte residual ratio is $10^{-3.7}$ and the red cell recovery is 89 %.

Comparative Example 12

A filter apparatus is prepared in substantially the same manner as in Example 6 except that the concentration of the copolymer in the ethanol solution is changed to 1.5 g/dl.

The molar ratio of the basic functional groups to the nonionic hydrophilic groups present in the surface of the filter material is 0.66, and the density of the basic functional groups present in the surface of the filter material is $1.6 \times 10^{-1}$ meq/m$^2$. The F values of the first filter material (having an average fiber diameter of 1.8 μm) and the second filter material (having an average fiber diameter of 1.2 μm) are, respectively, 2.0 g/sec/m and 1.0 g/sec/m. With respect to each of the first and second filter materials, the wetting time with pure water is less than one second.

The P value of the filter apparatus is 61.2 mmAq/g.

Using the filter apparatus, a filtration operation is conducted under substantially the same conditions as in Example 6.

When 320 ml of a red cell concentrate is subjected to filtration using the resultant filter apparatus, the flow rate of the red cell concentrate is lowered so drastically that only 30 ml of the red cell concentrate is recovered (the red cell recovery: 8.2 %). Accordingly, the determination of the leukocyte residual ratio is not possible. Furthermore, in the leukocyte-removed red cell concentrate, it is observed that hemolysis of erythrocytes occurred markedly.

Example 9

As prefilter materials for removing gels and macro- and micro-aggregates, three types of spun bonded non-woven fabrics made of polyester fibers respectively having average fiber diameters of 32 μm, 19 μm and 13 μm are packed in this order relative to the direction of blood flowing in a casing having a blood inlet and a blood outlet and having an effective filtration area of 67 mm x 67 mm so that the average packing density and total thickness of the three fabrics become 0.27 g/cm$^3$ and 1.8 mm, respectively. Then, as a first fibrous fabric for removing leukocytes, a polyester melt-blown non-woven fabric having an average fiber diameter of 1.7 μm is packed downstream of the above-mentioned prefilter materials for removing gels and macro- and micro-aggregates so that the packing density becomes 0.21 g/cm$^3$ and the thickness becomes 0.6 mm. Further, as a second fibrous fabric for removing leukocytes, a polyester melt-blown non-woven fabric having an average fiber diameter of 1.2 μm is packed downstream of the above-mentioned first fibrous fabric so that the packing density becomes 0.24 g/cm$^3$ and the thickness becomes 3.7 mm.

With respect to the subsequent procedure for preparing a filter apparatus, substantially the same procedure as in Example 6 is conducted except that the concentration of the copolymer in the ethanol solution is changed to 0.1 g/dl, to thereby obtain a filter apparatus comprising the casing and, packed therein, the prefilter materials for removing gels and macro- and micro-aggregates and the first and second filter materials for removing leukocytes.

The molar ratio of the basic functional groups to the nonionic hydrophilic groups present in the surface of the filter material is 0.66, and the density of the basic functional groups present in the surface of the filter material is $1.1 \times 10^{-2}$ meq/m$^2$. The F values of the first filter material (having an average fiber diameter of 1.7 μm) and the second filter material (having an average fiber diameter of 1.2 μm) are, respectively, 5.2 g/sec/m and 2.8 g/sec/m. With respect to each of the first and second filter materials, the wetting time with pure water is 3 seconds.

The P value of the filter apparatus is 16.8 mmAq/g.

Using the filter apparatus, a filtration operation is conducted under substantially the same conditions as in Example 1.

As a result, the leukocyte residual ratio is $10^{-5.9}$ and the red cell recovery is 90.2 %.

The time required for conducting filtration of the red cell concentrate is less than 25 minutes, and the flow rate of the red cell concentrate is good.

Example 10

As prefilter materials for removing gels and macro- and micro-aggregates, three types of spun bonded non-woven fabrics made of polyester fibers respectively having average fiber diameters of 32 μm, 19 μm and 13 μm are packed in this order relative to the direction of blood flowing in a casing having a blood inlet and a blood outlet and having an effective filtration area of 67 mm x 67 mm so that the average packing density and total thickness of the three fabrics become 0.26 g/cm$^3$ and 1.9 mm, respectively. Then, as a first fibrous fabric for removing leukocytes, a polyester melt-blown non-woven fabric having an average fiber diameter of 1.7 μm is packed downstream of the above-mentioned prefilter materials for removing gels and macro- and micro-aggregates so that the packing density becomes 0.2 g/cm$^3$ and the thickness becomes 0.6 mm. Further, as a second fibrous fabric for removing leukocytes, polyester melt-blown non-woven fabric having an average fiber diameter of 1.2 μm is packed downstream of the above-mentioned first fibrous fabric so that the packing density becomes 0.25 g/cm$^3$ and the thickness becomes 3.6 mm.

With respect to the subsequent procedure for preparing a filter apparatus, substantially the same procedure as in Example 6 is conducted except that the concentration of the copolymer in the ethanol solution is changed to 0.3 g/dl, to thereby obtain a filter apparatus comprising the casing and, packed therein, the prefilter materials for removing gels and macro- and micro-aggregates and the first and second filter materials for removing leukocytes.

The molar ratio of the basic functional groups to the nonionic hydrophilic groups present in the surface of the filter material is 0.66, and the density of the basic functional groups present in the surface of the filter material is $3.2 \times 10^{-2}$ meq/m$^2$. The F values of the first filter material (having an average fiber diameter of 1.7 μm) and the second filter material (having an average fiber diameter of 1.2 μm) are, respectively, 5.0 g/sec/m and 2.6 g/sec/m. With respect to each of the first and second filter materials, the wetting time with pure water is 3 seconds.

The P value of the filter apparatus is 17.6 mmAq/g.

Using the filter apparatus, a filtration operation is conducted under substantially the same conditions as in Example 1.

As a result, the leukocyte residual ratio is $10^{-6.2}$ and the red cell recovery is 91.3 %.

The time required for conducting filtration of the red cell concentrate is less than 25 minutes, and the flow rate of the red cell concentrate is good.

Example 11

As prefilter materials for removing gels and macro- and micro-aggregates, three types of spun bonded non-woven fabrics made of polyester fibers respectively having average fiber diameters of 32 μm, 19 μm and 13 μm are packed in this order relative to the direction of blood flowing in a casing having a blood inlet and a blood outlet and having an effective filtration area of 67 mm x 67 mm so that the average packing density and total thickness of the three fabrics become 0.27 g/cm$^3$ and 1.5 mm, respectively. Then, as a first fibrous fabric for removing leukocytes, a polyester melt-blown non-woven fabric having an average fiber diameter of 1.7 μm is packed downstream of the above-mentioned prefilter materials for removing gels and macro- and micro-aggregates so that the packing density becomes 0.22 g/cm$^3$ and the thickness becomes 0.6 mm. Further, as a second fibrous fabric for removing leukocytes, a polyester melt-blown non-woven fabric having an average fiber diameter of 1.2 μm is packed downstream of the above-mentioned first fibrous fabric so that the packing density becomes 0.28 g/cm$^3$ and the thickness becomes 4.0 mm.

With respect to the subsequent procedure for preparing a filter apparatus, substantially the same procedure as in Example 2 is conducted except that the concentration of the copolymer in the ethanol solution is changed to 0.1 g/dl, to thereby obtain a filter apparatus comprising the casing and, packed therein, the prefilter materials for removing gels and macro- and micro-aggregates and the first and second filter materials for removing leukocytes.

The molar ratio of the basic functional groups to the nonionic hydrophilic groups present in the surface of the filter material is 0.97, and the density of the basic functional groups present in the surface of the filter material is $1.2 \times 10^{-2}$ meq/m$^2$. The F values of the first filter material (having an average fiber diameter of 1.7 μm) and the second filter material (having an average fiber diameter of 1.2 μm) are, respectively, 5.2 g/sec/m and 2.5 g/sec/m. With respect to each of the first and second filter materials, the wetting time with pure water is 25 seconds.

The P value of the filter apparatus is 23.4 mmAq/g.

Using the filter apparatus, a filtration operation is conducted under substantially the same conditions as in Example 1.

As a result, the leukocyte residual ratio is $10^{-6.3}$ and the red cell recovery is 90.8 %.

The time required for conducting filtration of the red cell concentrate is less than 25 minutes, and the flow rate of the red cell concentrate is good.

Example 12

As prefilter materials for removing gels and macro- and micro-aggregates, three types of spun bonded non-woven fabrics made of polyester fibers respectively having average fiber diameters of 32 μm, 19 μm and 13 μm are packed in this order relative to the direction of blood flowing in a casing having a blood inlet and a blood outlet and having an effective filtration area of 67 mm x 67 mm so that the average packing density and total thickness of the three fabrics become 0.24 g/cm$^3$ and 2.0 mm, respectively. Then, as a first fibrous fabric for removing leukocytes, a polyester melt-blown non-woven fabric having an average fiber diameter of 1.7 μm is packed downstream of the above-mentioned prefilter materials for removing gels and macro- and micro-aggregates so that the packing density becomes 0.21 g/cm$^3$ and the thickness becomes 0.6 mm. Further, as a second fibrous fabric for removing leukocytes, a polyester melt-blown non-woven fabric having an average fiber diameter of 1.2 μm is packed into the downstream of the above-mentioned first fibrous fabric so that the packing density becomes 0.32 g/cm$^3$ and the thickness becomes 3.5 mm.

With respect to the subsequent procedure for preparing a filter apparatus, substantially the same procedure as in Example 6 is conducted except that the concentration of the copolymer in the ethanol solution is changed to 0.3 g/dl, to thereby obtain a filter apparatus comprising the casing and, packed therein, the prefilter materials for removing gels and macro- and micro-aggregates and the first and second filter materials for removing leukocytes.

The molar ratio of the basic functional groups to the nonionic hydrophilic groups present in the surface of the filter material is 0.97, and the density of the basic functional groups present in the surface of the filter material is $3.5 \times 10^{-2}$ meq/m$^2$. The F values of the first filter material (having an average fiber diameter of 1.7 µm) and the second filter material (having an average fiber diameter of 1.2 µm) are, respectively, 5.0 g/sec/m and 2.4 g/sec/m. With respect to each of the first and second filter materials, the wetting time with pure water is 28 seconds.

The P value of the filter apparatus is 25.8 mmAq/g.

Using the filter apparatus, a filtration operation is conducted under substantially the same conditions as in Example 1.

As a result, the leukocyte residual ratio is $10^{-6.7}$ and the red cell recovery is 92.2 %.

The time required for conducting filtration of the red cell concentrate is less than 25 minutes, and the flow rate of the red cell concentrate is good.

The results of Examples 6 to 12 and Comparative Examples 8 to 12 are shown in Table 2.

Table 2

| | Molar ratio of basic functional groups to nonionic hydrophilic groups | Surface basic functional group density (meq/m$^2$) | Leukocyte residual ratio | Red cell Recovery (%) |
|---|---|---|---|---|
| Example 6 | 0.66 | $1.1 \times 10^{-3}$ | $10^{-4.3}$ | 90.5 |
| Example 7 | 2.3 | $1.7 \times 10^{-3}$ | $10^{-4.3}$ | 90.0 |
| Example 8 | 5.7 | $2.0 \times 10^{-3}$ | $10^{-4.0}$ | 90.1 |
| Example 9 | 0.66 | $1.1 \times 10^{-2}$ | $10^{-5.9}$ | 90.2 |
| Example 10 | 0.66 | $3.2 \times 10^{-2}$ | $10^{-6.2}$ | 91.3 |
| Example 11 | 0.97 | $1.2 \times 10^{-2}$ | $10^{-6.3}$ | 90.8 |
| Example 12 | 0.97 | $3.5 \times 10^{-2}$ | $10^{-6.7}$ | 92.2 |
| Comparative Example 8 | - | - | $10^{-3.4}$ | 88.2 |
| Comparative Example 9 | 0.43 | $8.0 \times 10^{-4}$ | $10^{-3.6}$ | 90.4 |
| Comparative Example 10 | 7.7 | $2.2 \times 10^{-3}$ | $10^{-3.6}$ | 89.8 |
| Comparative Example 11 | 0.66 | $3.2 \times 10^{-5}$ | $10^{-3.7}$ | 89.0 |
| Comparative Example 12 | 0.66 | $1.6 \times 10^{-1}$ | - | 8.2 |

Example 13

Dimethylaminomethyl methacrylate (hereinafter referred to as "DM") and glycerol monomethacrylate (hereinafter referred to as "GMMA") are added to the same Wako special grade ethanol as mentioned above so that the final DM and GMMA concentrations of the resultant mixture become 0.6 mole/liter and 0.4 mole/liter, respectively, and the volume of the resultant mixture becomes 250 ml. To the obtained mixture is added V-65 as a polymerization initiator in a concentration of 0.05 mole/liter and then, polymerization is carried out at 45 °C for 4 hours in nitrogen atmosphere. The resultant reaction mixture is dropwise added to n-hexane to thereby obtain a precipitate. The obtained precipitate is collected and subjected to lyophilization to thereby obtain a copolymer of DM and GMMA.

With respect to the subsequent procedure for preparing a filter apparatus, substantially the same procedure as in Example 1 is conducted except that the first and second fibrous fabrics are coated with the copolymer of DM and GMMA, to thereby obtain a filter apparatus comprising the casing and the first and second filter materials packed therein.

The molar ratio of the basic functional groups to the nonionic hydrophilic groups present in the surface of the filter material is 0.75, and the density of the basic functional groups present in the surface of the filter material is $1.0 \times 10^{-3}$ meq/m$^2$. The F values of the first filter material (having an average fiber diameter of 1.8 µm) and the second filter material (having an average fiber diameter of 1.2 µm) are, respectively, 5.8 g/sec/m and 2.0 g/sec/m. With respect to each of the first and second filter materials, the wetting time with pure water is less than one second.

The P value of the filter apparatus is 13.7 mmAq/g.

Using the filter apparatus, a filtration operation is conducted under substantially the same conditions as in Example 1.

As a result, it is found that the leukocyte residual ratio is $10^{-4.5}$ and the red cell recovery is 92.0 %.

The time required for conducting filtration of the red cell concentrate is less than 25 minutes, and the flow rate of the red cell concentrate is good.

Example 14

DM and methoxytriethylene glycolmethacrylate (hereinafter referred to as "MTG") are added to the same Wako special grade ethanol as mentioned above so that the final DM and MTG concentrations of the resultant mixture become 0.6 mole/liter and 0.4 mole/liter, respectively, and the volume of the resultant mixture becomes 250 ml.

With respect to the subsequent procedure for preparing a filter apparatus, substantially the same procedure as in Example 1 is conducted except that the first and second fibrous fabrics are coated with the copolymer of DM and MTG, to thereby obtain a filter apparatus comprising the casing and the first and second filter materials packed therein.

The molar ratio of the basic functional groups to the nonionic hydrophilic groups present in the surface of the filter material is 1.6, and the density of the basic functional groups present in the surface of the filter material is $1.1 \times 10^{-3}$ meq/m$^2$. The F values of the first filter material (having an average fiber diameter of 1.8 $\mu$m) and the second filter material (having an average fiber diameter of 1.2 $\mu$m) are, respectively, 5.7 g/sec/m and 2.3 g/sec/m. With respect to each of the first and second filter materials, the wetting time with pure water is 4 seconds.

The P value of the filter apparatus is 14.2 mmAq/g.

Using the filter apparatus, a filtration operation is conducted under substantially the same conditions as in Example 1.

As a result, it is found that the leukocyte residual ratio is $10^{-4.7}$ and the red cell recovery is 93.1 %.

The time required for conducting filtration of the red cell concentrate is less than 25 minutes, and the flow rate of the red cell concentrate is good.

Example 15

DM, MTG and methyl methacrylate (hereinafter referred to as "MMA") are added to the same Wako special grade ethanol as mentioned above so that the final DM, MTG and MMA concentrations of the resultant mixture become 0.4 mole/liter, 0.4 mole/liter and 0.2 mole/liter, respectively, and the volume of the resultant mixture becomes 250 ml.

With respect to the subsequent procedure for preparing a filter apparatus, substantially the same procedure as in Example 1 is conducted except that the first and second fibrous fabrics are coated with the copolymer of DM, MTG and MMA, to thereby obtain a filter apparatus comprising the casing and the first and second filter materials packed therein.

The molar ratio of the basic functional groups to the nonionic hydrophilic groups present in the surface of the filter material is 1.2, and the density of the basic functional groups present in the surface of the filter material is $7.6 \times 10^{-4}$ meq/m$^2$. The F values of the first filter material (having an average fiber diameter of 1.8 $\mu$m) and the second filter material (having an average fiber diameter of 1.2 $\mu$m) are, respectively, 5.9 g/sec/m and 2.0 g/sec/m. With respect to each of the first and second filter materials, the wetting time with pure water is 18 seconds.

The P value of the filter apparatus is 14.0 mmAq/g.

Using the filter apparatus, a filtration operation is conducted under substantially the same conditions as in Example 1.

As a result, it is found that the leukocyte residual ratio is $10^{-4.8}$ and the red cell recovery is 91.6 %.

The time required for conducting filtration of the red cell concentrate is less than 25 minutes, and the flow rate of the red cell concentrate is good.

Example 16

DM and methoxyoctaethylene glycolmethacrylate (hereinafter referred to as "MOG") are added to the same Wako special grade ethanol as mentioned above so that the final DM and MOG concentrations of the resultant mixture become 0.6 mole/liter and 0.4 mole/liter, respectively, and the volume of the resultant mixture becomes 250 ml.

With respect to the subsequent procedure for preparing a filter apparatus, substantially the same procedure as in Example 1 is conducted except that the first and second fibrous fabrics are coated with the copolymer of DM and MOG, to thereby obtain a filter apparatus comprising the casing and the first and second filter materials packed therein.

The molar ratio of the basic functional groups to the nonionic hydrophilic groups present in the surface of the filter material is 0.77, and the density of the basic functional groups present in the surface of the filter material is $7.3 \times 10^{-4}$ meq/m$^2$. The F values of the first filter material (having an average fiber diameter of 1.8 $\mu$m) and the second filter material (having an average fiber diameter of 1.2 $\mu$m) are, respectively, 5.7 g/sec/m and 2.6 g/sec/m. With respect to each of the first and second filter materials, the wetting time with pure water is less than one second.

The P value of the filter apparatus is 14.5 mmAq/g.

Using the filter apparatus, a filtration operation is conducted under substantially the same conditions as in Example 1.

As a result, it is found that the leukocyte residual ratio is $10^{-4.5}$ and the red cell recovery is 91.3 %.

The time required for conducting filtration of the red cell concentrate is less than 25 minutes, and the flow rate of the red cell concentrate is good.

Example 17

DMA and methoxypolyethylene glycolmethacrylate (hereinafter referred to as "MPG") in which the number of the recurring ethylene oxide unit is 30 are added to the same Wako special grade ethanol as mentioned above so that the final DM and MPG concentrations of the resultant mixture become 0.4 mole/liter and 0.6 mole/liter, respectively, and the volume of the resultant mixture becomes 250 ml. To the obtained mixture is added V-65 as a polymerization initiator in a concentration of 0.05 mole/liter and then, polymerization is carried out at 45 °C for 2.5 hours in nitrogen atmosphere. The resultant reaction mixture is dropwise added to n-hexane to thereby obtain a precipitate. The obtained precipitate is collected and subjected to lyophilization to thereby obtain a copolymer of DM and MPG.

With respect to the subsequent procedure for preparing a filter apparatus, substantially the same procedure as in Example 6 is conducted except that the prefilter materials and the first and second fibrous fabrics are coated with the copolymer of DM and MPG, to thereby obtain a filter apparatus comprising the casing and, packed therein, the prefilter materials for removing gels and macro- and micro-aggregates, the prefilter materials for removing microaggregates and the first and second filter materials for removing leukocytes.

The molar ratio of the basic functional groups to the nonionic hydrophilic groups present in the surface of the filter material is 0.7, and the density of the basic functional groups present in the surface of the filter material is $1.5 \times 10^{-4}$ meq/m$^2$. The F values of the first filter material (having an average fiber diameter of 1.8 $\mu$m) and the second filter material (having an average fiber diameter of 1.2 $\mu$m) are, respectively, 5.5 g/sec/m and 2.9 g/sec/m. With respect to each of the first and second filter materials, the wetting time with pure water is 9 seconds.

The P value of the filter apparatus is 13.9 mmAq/g.

Using the filter apparatus, a filtration operation is conducted under substantially the same conditions as in Example 6, except that a red cell concentrate which has been stored at 4 °C for 12 days (hematocrit: 65 %) is used.

As a result, it is found that the leukocyte residual ratio is $10^{-4.0}$ and the red cell recovery is 90.3 %.

The time required for conducting filtration of the red cell concentrate is less than 25 minutes, and the flow rate of the red cell concentrate is good.

The results of the above Examples 13 to 17 are shown in Table 3.

Table 3

|  | Molar ratio of basic functional groups to nonionic hydrophilic groups | Surface basic functional group density (meq/m$^2$) | Leukocyte residual ratio | Red cell recovery (%) |
|---|---|---|---|---|
| Example 13 | 0.75 | $1.0 \times 10^{-3}$ | $10^{-4.5}$ | 92.0 |
| Example 14 | 1.6 | $1.1 \times 10^{-3}$ | $10^{-4.7}$ | 93.1 |
| Example 15 | 1.2 | $7.6 \times 10^{-4}$ | $10^{-4.8}$ | 91.6 |
| Example 16 | 0.77 | $7.3 \times 10^{-4}$ | $10^{-4.5}$ | 91.3 |
| Example 17 | 0.7 | $1.5 \times 10^{-4}$ | $10^{-4.0}$ | 90.3 |

For easy reference, the results of all of Examples 1 to 17 and Comparative Examples 1 to 12 are shown in Tables 4(a) through 4(b).

## Table 4 (a)

| Example Nos. | Comparative Example Nos. | Ratio of polymers constituting copolymer | Leukocyte residual ratio (%) | Red cell reco-very (%) | Molar ratio of basic functional groups to non-ionic hydrophil-ic groups | Surface basic functional group density ($\times 10^{-5}$ meq/m$^2$) | Concentration of copolymer in ethanol (g/dl) |
|---|---|---|---|---|---|---|---|
| 1 | | DM40/HEMA60 | $10^{-4.8}$ | 91.9 | 0.67 | 95 | 0.01 |
| | 1 | — | $10^{-3.7}$ | 90.6 | — | — | — |
| 2 | | DM50/HEMA50 | $10^{-5}$ | 92.7 | 0.97 | 120 | 0.01 |
| 3 | | DM60/HEMA40 | $10^{-5.2}$ | 92.0 | 1.49 | 140 | 0.01 |
| 4 | | DM70/HEMA30 | $10^{-5.2}$ | 91.8 | 2.31 | 160 | 0.01 |
| 5 | | DM80/HEMA20 | $10^{-5}$ | 90.5 | 3.98 | 180 | 0.01 |
| | 2 | DE20/HEMA80 | $10^{-3.8}$ | 93.0 | 0.25 | 47 | 0.01 |
| | 3 | DM30/HEMA70 | $10^{-3.9}$ | 92.3 | 0.43 | 72 | 0.01 |
| | 4 | DM90/HEMA10 | $10^{-3.6}$ | 91.1 | 8.94 | 200 | 0.01 |
| | 5 | DE100 | $10^{-3.8}$ | 89.7 | — | 180 | 0.01 |
| | 6 | DM50/HEMA50 | $10^{-3.8}$ | 90.9 | 0.97 | 3.5 | 0.0003 |
| | 7 | DM50/HEMA50 | — | 16.3 | 0.97 | 12000 | 1 |
| 6 | | DMA40/HEA60 | $10^{-4.3}$ | 90.5 | 0.66 | 110 | 0.01 |
| 7 | | DMA70/HEA30 | $10^{-4.3}$ | 90.0 | 2.3 | 170 | 0.01 |
| 8 | | DMA85/HEA15 | $10^{-4}$ | 90.1 | 5.7 | 200 | 0.01 |
| | 8 | — | $10^{-3.4}$ | 88.2 | — | — | — |
| | 9 | DMA30/HEA70 | $10^{-3.6}$ | 90.4 | 0.43 | 80 | 0.01 |
| | 10 | DMA90/HEA10 | $10^{-3.6}$ | 89.8 | 7.7 | 220 | 0.01 |
| | 11 | DMA40/HEA60 | $10^{-3.7}$ | 89.0 | 0.66 | 3.2 | 0.0003 |
| | 12 | DMA40/HEA60 | — | 8.2 | 0.66 | 16000 | 1.5 |
| 9 | | DMA40/HEA60 | $10^{-5.9}$ | 90.2 | 0.66 | 1100 | 0.1 |
| 10 | | DMA40/HEA60 | $10^{-6.2}$ | 91.3 | 0.66 | 3200 | 0.3 |
| 11 | | DM50/HEMA50 | $10^{-6.3}$ | 90.8 | 0.97 | 1200 | 0.1 |
| 12 | | DM50/HEMA50 | $10^{-6.7}$ | 92.2 | 0.97 | 3500 | 0.3 |
| 13 | | DM60/GMMA40 | $10^{-4.5}$ | 92.0 | 0.75 | 100 | 0.01 |
| 14 | | DM60/MTG40 | $10^{-4.7}$ | 93.1 | 1.6 | 110 | 0.01 |
| 15 | | DM40/MTG40/MMA20 | $10^{-4.8}$ | 91.6 | 1.2 | 76 | 0.01 |
| 16 | | DM60/MOG40 | $10^{-4.5}$ | 91.3 | 0.77 | 73 | 0.01 |
| 17 | | DMA40/MPG60 | $10^{-4}$ | 90.3 | 0.7 | 15 | 0.01 |

24

## Table 4 (b)

| Ex. Nos. | Comp. Ex. Nos. | Average fiber diameter (μm) | | Packing density (g/cm3) | | Thickness (mm) | | Wetting time with pure water (sec) | F value * (g/sec/m) | | P value** of filter apparatus (mmAq/g) |
|---|---|---|---|---|---|---|---|---|---|---|---|
| | | 1st Filter material | 2nd Filter material | 1st Filter material | 2nd Filter material | 1st Filter material | 2nd Filter material | | 1st Filter material | 2nd Filter material | |
| 1 | | 1.8 | 1.2 | 0.18 | 0.2 | 1.5 | 3 | 16 | 5.6 | 3.3 | 13.8 |
| | 1 | 1.8 | 1.2 | 0.18 | 0.2 | 1.5 | 3 | >60 | 5.7 | 3.2 | 14.7 |
| 2 | | 1.8 | 1.2 | 0.18 | 0.2 | 1.5 | 3 | 19 | 5.5 | 3.3 | 14.2 |
| 3 | | 1.8 | 1.2 | 0.18 | 0.2 | 1.5 | 3 | 20 | 5.4 | 3.0 | 15.1 |
| 4 | | 1.8 | 1.2 | 0.18 | 0.2 | 1.5 | 3 | 22 | 5.4 | 3.1 | 14.3 |
| 5 | | 1.8 | 1.2 | 0.18 | 0.2 | 1.5 | 3 | 22 | 5.6 | 3.5 | 13.9 |
| | 2 | 1.8 | 1.2 | 0.18 | 0.2 | 1.5 | 3 | 11 | 5.3 | 3.3 | 14.9 |
| | 3 | 1.8 | 1.2 | 0.18 | 0.2 | 1.5 | 3 | 13 | 5.6 | 3.6 | 15.3 |
| | 4 | 1.8 | 1.2 | 0.18 | 0.2 | 1.5 | 3 | 38 | 5.8 | 3.4 | 14.2 |
| | 5 | 1.8 | 1.2 | 0.18 | 0.2 | 1.5 | 3 | 45 | 5.5 | 3.4 | 14.7 |
| | 6 | 1.8 | 1.2 | 0.18 | 0.2 | 1.5 | 3 | 19 | 6.0 | 3.5 | 14.5 |
| | 7 | 1.8 | 1.2 | 0.18 | 0.2 | 1.5 | 3 | 23 | 2.5 | 1.3 | 53.0 |
| 6 | | 1.8 | 1.2 | 0.18 | 0.2 | 1.5 | 3 | <1 | 5.5 | 3.3 | 14.3 |
| 7 | | 1.8 | 1.2 | 0.18 | 0.2 | 1.5 | 3 | 8 | 5.6 | 3.3 | 14.7 |
| 8 | | 1.8 | 1.2 | 0.18 | 0.2 | 1.5 | 3 | 15 | 5.6 | 3.5 | 15 |
| | 8 | 1.8 | 1.2 | 0.18 | 0.2 | 1.5 | 3 | >60 | 5.6 | 3.6 | 15.1 |
| | 9 | 1.8 | 1.2 | 0.18 | 0.2 | 1.5 | 3 | <1 | 5.5 | 3.7 | 13.9 |
| | 10 | 1.8 | 1.2 | 0.18 | 0.2 | 1.5 | 3 | 16 | 5.7 | 3.5 | 14.8 |
| | 11 | 1.8 | 1.2 | 0.18 | 0.2 | 1.5 | 3 | <1 | 5.4 | 3.4 | 13.8 |
| | 12 | 1.8 | 1.2 | 0.18 | 0.2 | 1.5 | 3 | <1 | 2.0 | 1.0 | 61.2 |
| 9 | | 1.7 | 1.2 | 0.21 | 0.24 | 0.6 | 3.7 | 3 | 5.2 | 2.8 | 16.8 |
| 10 | | 1.7 | 1.2 | 0.2 | 0.25 | 0.6 | 3.6 | 3 | 5.0 | 2.6 | 17.6 |
| 11 | | 1.7 | 1.2 | 0.22 | 0.28 | 0.6 | 4 | 25 | 5.2 | 2.5 | 23.4 |
| 12 | | 1.7 | 1.2 | 0.21 | 0.32 | 0.6 | 3.5 | 28 | 5.0 | 2.4 | 25.8 |
| 13 | | 1.8 | 1.2 | 0.18 | 0.2 | 1.5 | 3 | <1 | 5.8 | 2.0 | 13.7 |
| 14 | | 1.8 | 1.2 | 0.18 | 0.2 | 1.5 | 3 | 4 | 5.7 | 2.3 | 14.2 |
| 15 | | 1.8 | 1.2 | 0.18 | 0.2 | 1.5 | 3 | 18 | 5.9 | 2.0 | 14 |
| 16 | | 1.8 | 1.2 | 0.18 | 0.2 | 1.5 | 3 | <1 | 5.7 | 2.6 | 14.5 |
| 17 | | 1.8 | 1.2 | 0.18 | 0.2 | 1.5 | 3 | 9 | 5.5 | 2.9 | 13.9 |

* The F value = Q x R x 100

> wherein Q represents the air permeability of the non-woven fabric $(cm^3/sec/cm^2)$ and R represents the weight of the non-woven fabric per $cm^2$ $(g/cm^2)$

$$** \text{ The P value } = \frac{\text{pressure loss of the air passing through the filter apparatus } (mmAq)}{\text{weight of the effective filtration area of the filter material having an average fiber diameter of 0.3 to 5 } \mu m \text{ (g)}}$$

The first fibrous fabric and the second fibrous fabric used in Comparative Examples 1 and 8 are not coated with copolymer, but they are referred to as "1st filter material" and "2nd filter material" in Table 4 (b) above, respectively, for the sake of convenience.

**Claims**

1. A filter material for removing leukocytes from a whole blood product or a red cell product, comprising at least one layer of a porous element having a three-dimensional network of continuous open pores, and wherein said porous element has, in a surface portion thereof, basic functional groups at a density of from $5 \times 10^{-5}$ to 0.1 meq/m$^2$ and nonionic hydrophilic groups, wherein a molar ratio of said basic functional groups to said nonionic hydrophilic groups is in the range from 0.6 to 6.

2. The filter material according to claim 1, wherein said porous element has an F value of from 1.6 to 10 g/second/m, said F value being defined by the following formula:

$$F \text{ value} = Q \times R \times 100$$

wherein Q represents the air permeability of porous element $(cm^3/sec/cm^2)$ and R represents the weight of the porous element per cm$^2$ $(g/cm^2)$, and wherein the air permeability is the volume (cm$^3$) of the air passing through the porous element per second per cm$^2$ of the porous element as measured at 20 °C at a pressure difference of 12.7 mmAq between opposite sides of the porous element in accordance with Japanese Industrial Standard (JIS) L-1096.

3. The filter material according to claim 2 or 3, wherein said porous element is selected from the group consisting of a fibrous fabric and a non-fibrous porous structure.

4. The filter material according to claim 3, wherein said fibrous fabric is a non-woven fabric comprising fibers having an average fiber diameter of from 0.3 to 5 $\mu$m.

5. The filter material according to any one of claims 1 to 4, wherein said basic functional groups are tertiary amino groups.

6. The filter material according to any one of claims 1 to 5, wherein said nonionic hydrophilic groups are selected from the group consisting of hydroxyl groups, polyethylene oxide groups and a mixture thereof.

7. A filter apparatus for removing leukocytes from a whole blood product or a red cell product, which comprises a casing having a blood inlet and a blood outlet, and a filter material accommodated in said casing between said blood inlet and said blood outlet,

> said filter material comprising at least one layer of a porous element having a three-dimensional network of continuous open pores, and wherein said porous element has, in a surface portion thereof, basic functional groups at a density of from 5 x 10$^{-5}$ to 0.1 meq/m$^2$ and nonionic hydrophilic groups, wherein a molar ratio of said basic functional groups to said nonionic hydrophilic groups is in the range from 0.6 to 6,

said filter apparatus having a P value of from 3 to 50 mmAq/g, said P value being defined by the following formula:

$$P\ value = \frac{pressure\ loss\ of\ the\ air\ passing\ through\ the\ filter\ apparatus\ (mmAq)}{weight\ of\ the\ effective\ filtration\ area\ of\ the\ filter\ material\ (g)}$$

wherein the pressure loss of the air passing through the filter apparatus is the pressure difference as measured between the blood inlet and the blood outlet under conditions such that dry air is passed through the filter apparatus from the blood inlet of the apparatus to the blood outlet of the apparatus at a constant flow rate of 3 liters/minute at room temperature.

8. The filter apparatus according to claim 7, wherein said porous element has an F value of from 1.6 to 10 g/second/m, said F value being defined by the following formula:

$$F\ value = Q\ x\ R\ x\ 100$$

wherein Q represents the air permeability of porous element (cm$^3$/sec/cm$^2$) and R represents the weight of the porous element per cm$^2$ (g/cm$^2$), and wherein the air permeability is the volume (cm$^3$) of the air passing through the porous element per second per cm$^2$ of the porous element as measured at 20 °C at a pressure difference of 12.7 mmAq between opposite sides of the porous element in accordance with Japanese Industrial Standard (JIS) L-1096.

9. The filter apparatus according to claim 7 or 8, wherein said porous element is selected from the group consisting of a fibrous fabric and a non-fibrous porous structure.

10. The filter apparatus according to claim 9, wherein said fibrous fabric is a non-woven fabric comprising fibers having an average fiber diameter of from 0.3 to 5 μm.

11. The filter apparatus according to any one of claims 7 to 10, wherein said filter material has an S/T value of from 50 to 300 cm in terms of the quotient of the effective filtration area (S) (cm$^2$) divided by the thickness (T) (cm) thereof.

12. The filter apparatus according to any one of claims 7 to 11, wherein said filter material comprises a plurality of porous element layers having different average pore diameters, wherein said filter material has a gradient with respect to the average pore diameter such that the average pore diameter is substantially continuously or stepwise decreased from an upstream end portion to a downstream end portion of the filter material in a flow direction in which a whole blood product or a red cell product is adapted to be flowed and wherein a porous element layer which is present upstream of the downstream end portion of said filter material in said flow direction has an average pore diameter which is at least 1.2 times that of a porous element layer present at the downstream end portion of said filter material.

13. The filter apparatus according to any one of claims 7 to 12, wherein said basic functional groups are tertiary amino groups.

14. The filter apparatus according to any one of claims 7 to 13, wherein said nonionic hydrophilic groups are selected from the group consisting of hydroxyl groups, polyethylene oxide groups and a mixture thereof.

15. A method for removing leukocytes from a whole blood product or a red cell product, comprising subjecting a whole blood product or a red cell product to filtration with a filter apparatus,

said filter apparatus comprising a casing having a blood inlet and a blood outlet, and a filter material accommodated in said casing between said blood inlet and said blood outlet,

said filter material comprising at least one layer of a porous element having a three-dimensional network of continuous open pores, and wherein said porous element has, in a surface portion thereof, basic functional groups at a density of from 5 x 10$^{-5}$ to 0.1 meq/m$^2$ and nonionic hydrophilic groups, wherein a molar ratio of said basic functional groups to said nonionic hydrophilic groups is in the range from 0.6 to 6,

said filter apparatus having a P value of from 3 to 50 mmAq/g, said P value being defined by the following for-

mula:

$$P \text{ value} = \frac{\text{pressure loss of the air passing through the filter apparatus (mmAq)}}{\text{weight of the effective filtration area of the filter material (g)}}$$

wherein the pressure loss of the air passing through the filter apparatus is the pressure difference as measured between the blood inlet and the blood outlet under conditions such that dry air is passed through the filter apparatus from the blood inlet of the apparatus to the blood outlet of the apparatus at a constant flow rate of 3 liters/minute at room temperature,

said a whole blood product or red cell product being treated in an amount of 0.1 to 0.5 unit per g of said filter material.

16. The method according to claim 15, wherein said porous element has an F value of from 1.6 to 10 g/second/m, said F value being defined by the following formula:

$$F \text{ value} = Q \times R \times 100$$

wherein Q represents the air permeability of porous element ($cm^3/sec/cm^2$) and R represents the weight of the porous element per $cm^2$ ($g/cm^2$), and wherein the air permeability is the volume ($cm^3$) of the air passing through the porous element per second per $cm^2$ of the porous element as measured at 20 °C at a pressure difference of 12.7 mmAq between opposite sides of the porous element in accordance with Japanese Industrial Standard (JIS) L-1096.

17. The method according to claim 15 or 16, wherein said porous element is selected from the group consisting of a fibrous fabric and a non-fibrous porous structure.

18. The method according to claim 17, wherein said fibrous fabric is a non-woven fabric comprising fibers having an average fiber diameter of from 0.3 to 5 $\mu$m.

19. The method according to any one of claims 15 to 18, wherein said filter material has an S/T value of from 50 to 300 cm in terms of the quotient of the effective filtration area (S) ($cm^2$) divided by the thickness (T) (cm) thereof.

20. The method according to any one of claims 15 to 19, wherein said filter material comprises a plurality of porous element layers having different average fiber diameters, wherein said filter material has a gradient with respect to the average pore diameter such that the average pore diameter is substantially continuously or stepwise decreased from an upstream end portion to a downstream end portion of the filter material in a flow direction in which a whole blood product or a red cell product is adapted to be flowed and wherein a porous element layer which is present upstream of the downstream end portion of said filter material in said flow direction has an average pore diameter which is at least 1.2 times that of a porous element layer present at the downstream end portion of said filter material.

21. The method according to any one of claims 15 to 20, wherein said basic functional groups are tertiary amino groups and said nonionic hydrophilic groups are selected from the group consisting of hydroxyl groups, polyethylene oxide groups and a mixture thereof.

**Patentansprüche**

1. Filtermaterial zur Entfernung von Leukocyten aus einem Vollblutprodukt oder einem rote Blutzellen enthaltenden Produkt, welches mindestens eine Schicht eines porösen Elements mit einem dreidimensionalen Netzwerk von durchgehenden offenen Poren umfaßt, wobei das poröse Element in einem seiner Oberflächenbereiche basische funktionelle Gruppen in einer Dichte von 5 x $10^{-5}$ bis 0,1 meq/$m^2$ und nicht-ionische hydrophile Gruppen aufweist, wobei das molare Verhältnis der basischen funktionellen Gruppen zu den nicht-ionischen hydrophilen Gruppen im Bereich von 0,6 bis 6 liegt.

2. Filtermaterial gemäß Anspruch 1, wobei das poröse Element einen F-Wert von 1,6 bis 10 g/s/m aufweist, wobei der F-Wert durch die folgende Formel definiert ist:

$$F\text{-Wert} = Q \times R \times 100$$

worin Q die Luftdurchlässigkeit des porösen Elements ($cm^3$/s/$cm^2$) und R das Gewicht des porösen Elements pro $cm^2$ (g/$cm^2$) darstellt, und worin die Luftdurchlässigkeit das Volumen ($cm^3$) der Luft ist, die das poröse Element pro Sekunde pro $cm^2$ des porösen Elements passiert, wobei die Luftdurchlässigkeit bei 20°C bei einer Druckdifferenz von 12,7 mmAq zwischen den gegenüberliegenden Seiten des porösen Elements gemäß Japanese Industrial Standard (JIS) L-1096 gemessen wird.

3. Filtermaterial gemäß Anspruch 2 oder 3, worin das poröse Element aus der aus Faserstoff und einer nicht-faserförmigen porösen Struktur bestehenden Gruppe ausgewählt ist.

4. Filtermaterial gemäß Anspruch 3, worin der Faserstoff ein Faservlies ist, das Fasern mit einem durchschnittlichen Faserdurchmesser von 0,3 bis 5 $\mu$m enthält.

5. Filtermaterial gemäß einem der Ansprüche 1 bis 4, worin die basischen funktionellen Gruppen tertiäre Aminogruppen sind.

6. Filtermaterial gemäß einem der Ansprüche 1 bis 5, worin die nicht-ionischen hydrophilen Gruppen aus der aus Hydroxylgruppen, Polyethylenoxidgruppen und einem Gemisch davon bestehenden Gruppe ausgewählt sind.

7. Filtervorrichtung zur Entfernung von Leukocyten aus einem Vollblutprodukt oder einem rote Blutzellen enthaltenden Produkt, welches ein Gehäuse mit einem Bluteinlaß und einem Blutauslaß und ein Filtermaterial, das in dem Gehäuse zwischen dem Bluteinlaß und dem Blutauslaß eingepaßt ist, aufweist, wobei das Filtermaterial mindestens eine Schicht eines porösen Elements mit einem dreidimensionalen Netzwerk von durchgehenden offenen Poren umfaßt, und wobei das poröse Element in einem seiner Oberflächenbereiche basische funktionelle Gruppen in einer Dichte von 5 x $10^{-5}$ bis 0,1 meq/$m^2$ und nicht-ionischen hydrophile Gruppen hat, wobei das molare Verhältnis der basischen funktionellen Gruppen zu den nicht-ionischen hydrophilen Gruppen in dem Bereich von 0,6 bis 6 liegt, und die Filtervorrichtung einen P-Wert von 3 bis 50 mmAq/g hat, wobei der P-Wert durch die folgende Formel definiert ist:

$$\text{P-Wert} = \frac{\text{Druckverlust der Luft, die die Filtervorrichtung passiert (mmAq)}}{\text{Gewicht der effektiven Filterfläche des Filtermaterials (g)}}$$

worin der Druckverlust der Luft, die die Filtervorrichtung passiert, die Druckdifferenz ist, die zwischen dem Bluteinlaß und dem Blutauslaß unter der Bedingung gemessen wird, daß trockene Luft die Filtervorrichtung vom Bluteinlaß der Vorrichtung zum Blutauslaß der Vorrichtung bei einer konstanten Flußrate von 3 Liter/Minute bei Raumtemperatur passiert.

8. Filtervorrichtung gemäß Anspruch 7, worin das poröse Element einen F-Wert von 1,6 bis 10 g/s/m aufweist, wobei der F-Wert in der folgenden Formel definiert ist:

$$\text{F-Wert} = Q \times R \times 100$$

worin Q die Luftdurchlässigkeit des porösen Elements ($cm^3$/s/$cm^2$) und R das Gewicht des porösen Elements pro $cm^2$ (g/$cm^2$) darstellt, und wobei die Luftdurchlässigkeit das Volumen ($cm^3$) der Luft ist, die das poröse Element pro Sekunde pro $cm^2$ des porösen Elements passiert und die Luftdurchlässigkeit bei 20°C bei einer Druckdifferenz von 12,7 mmAq zwischen den gegenüberliegenden Seiten des porösen Elements gemäß Japanese Industrial Standard (JIS) L-1096 gemessen wird.

9. Filtervorrichtung gemäß Anspruch 7 oder 8, worin das poröse Element aus der aus einem Faserstoff und einer nicht-faserförmigen porösen Struktur bestehenden Gruppe ausgewählt ist.

10. Filtervorrichtung gemäß Anspruch 9, worin der Faserstoff ein Faservlies ist, das Fasern mit einem durchschnittlichen Faserdurchmesser von 0,3 bis 5 $\mu$m enthält.

11. Filtervorrichtung gemäß einem der Ansprüche 7 bis 10, worin das Filtermaterial einen S/T-Wert von 50 bis 300 cm, ausgedrückt als der Quotient der effektiven Filterfläche (S) ($cm^2$) geteilt durch deren Dicke (T) (cm), hat.

12. Filtervorrichtung gemäß einem der Ansprüche 7 bis 11, worin das Filtermaterial eine Vielzahl von Schichten aus porösen Elementen mit unterschiedlichen durchschnittlichen Porendurchmessern enthält, wobei das Filtermaterial einen solchen Gradienten in bezug auf den durchschnittlichen Porendurchmesser aufweist, daß der durchschnittliche Porendurchmesser von einem stromaufwärts liegenden Endbereich zu einem stromabwärts liegenden Endbereich des Filtermaterials in der Flußrichtung, in welcher man das Vollblutprodukt oder das rote Blutzellen enthaltende Produkt fließen läßt, im wesentlichen stetig oder schrittweise abnimmt, und worin eine Schicht eines porösen Elements, die stromaufwärts des stromabwärts liegenden Endbereichs des Filtermaterials in der Flußrichtung vorhanden ist, einen durchschnittlichen Porendurchmesser aufweist, der mindestens das 1,2fache des Porendurchmessers der Schicht des porösen Elements beträgt, die an dem stromabwärts liegenden Endbereich des Filtermaterials vorhanden ist.

13. Filtervorrichtung gemäß einem der Ansprüche 7 bis 12, worin die basischen funktionellen Gruppen tertiäre Aminogruppen sind.

14. Filtervorrichtung gemäß einem der Ansprüche 7 bis 13, worin die nicht-ionischen hydrophilen Gruppen aus der aus Hydroxylgruppen, Polyethylenoxidgruppen und einem Gemisch davon bestehenden Gruppe ausgewählt sind.

15. Verfahren zur Entfernung von Leukocyten aus einem Vollblutprodukt oder einem rote Blutzellen enthaltenden Produkt, welches die Filtration des Vollblutprodukts oder des rote Blutzellen enthaltenden Produkts mit einer Filtervorrichtung umfaßt,

wobei die Filtervorrichtung ein Gehäuse mit einem Bluteinlaß und einem Blutauslaß und ein Filtermaterial aufweist, das in dem Gehäuse zwischen dem Bluteinlaß und dem Blutauslaß einpaßt ist,
wobei das Filtermaterial mindestens eine Schicht eines porösen Elements mit einem dreidimensionalen Netzwerk von durchgehenden offenen Poren enthält, und wobei das poröse Element in einem seiner Oberflächenbereiche basische funktionelle Gruppen in einer Dichte von $5 \times 10^{-5}$ bis 0,1 meq/m$^2$ und nicht-ionische hydrophile Gruppen aufweist, wobei das molare Verhältnis der basischen funktionellen Gruppen zu den nicht-ionischen hydrophilen Gruppen im Bereich von 0,6 bis 6 liegt, wobei die Filtervorrichtung einen P-Wert von 3 bis 50 mmAq/g aufweist, wobei der P-Wert in der folgenden Formel definiert ist:

$$\text{P-Wert} = \frac{\text{Druckverlust der Luft, die die Filtervorrichtung passiert (mmAq)}}{\text{Gewicht der effektiven Filterfläche des Filtermaterials (g)}}$$

wobei der Druckverlust der Luft, die die Filtervorrichtung passiert, die Druckdifferenz ist, die zwischen dem Bluteinlaß und dem Blutauslaß unter der Bedingung gemessen wird, daß trockene Luft die Filtervorrichtung von dem Bluteinlaß der Vorrichtung zu dem Blutauslaß der Vorrichtung bei einer konstanten Flußrate von 3 Liter/Minute bei Raumtemperatur passiert,
wobei ein Vollblutprodukt oder ein rote Blutzellen enthaltendes Produkt in einer Menge von 0,1 bis 0,5 Einheit pro g des Filtermaterials behandelt wird.

16. Verfahren gemäß Anspruch 15, wobei das poröse Element einen F-Wert von 1,6 bis 10 g/s/m aufweist, wobei der F-Wert durch folgende Formel definiert ist:

$$\text{F-Wert} = Q \times R \times 100$$

worin Q die Luftdurchlässigkeit des porösen Elements (cm$^3$/s/cm$^2$) und R das Gewicht des porösen Elements pro cm$^2$ (g/cm$^2$) darstellt, wobei die Luftdurchlässigkeit das Volumen (cm$^3$) der Luft ist, die das poröse Element pro Sekunde pro cm$^2$ des porösen Elements passiert, wobei die Luftdurchlässigkeit bei 20°C bei einer Druckdifferenz von 12,7 mmAq zwischen den gegenüberliegenden Seiten des porösen Elements gemäß Japanese Industrial Standard (JIS) L-1096 gemessen wird.

17. Verfahren gemäß Anspruch 15 oder 16, wobei das poröse Element aus der aus einem Faserstoff und einer nicht-faserförmigen porösen Struktur bestehenden Gruppe ausgewählt ist.

18. Verfahren gemäß Anspruch 17, wobei der Faserstoff ein Faservlies ist, das Fasern mit einem durchschnittlichen Faserdurchmesser von 0,3 bis 5 μm enthält.

19. Verfahren gemäß einem der Ansprüche 15 bis 18, wobei das Filtermaterial einen S/T-Wert von 50 bis 300 cm, ausgedrückt als der Quotient der effektiven Filterfläche (S) (cm$^2$) geteilt durch deren Dicke (T) (cm), hat.

**20.** Verfahren gemäß einem der Ansprüche 15 bis 19, wobei das Filtermaterial eine Vielzahl von Schichten aus porösen Elementen mit unterschiedlichen durchschnittlichen Faserdurchmessern enthält, wobei das Filtermaterial einen solchen Gradienten in bezug auf den durchschnittlichen Porendurchmesser aufweist, daß der durchschnittliche Porendurchmesser von einem stromaufwärts liegenden Endbereich zu einem stromabwärts liegenden Endbereich des Filtermaterials in der Flußrichtung, in der man ein Vollblutprodukt oder ein rote Blutzellen enthaltendes Produkt fließen läßt, im wesentlichen stetig oder schrittweise abnimmt, wobei eine Schicht eines porösen Elements, die stromaufwärts von dem stromabwärts liegenden Endbereich des Filtermaterials in der Flußrichtung vorhanden ist, einen durchschnittlichen Porendurchmesser aufweist, der mindestens das 1,2fache des Porendurchmessers einer Schicht eines porösen Elements aufweist, die in dem stromabwärts liegenden Endbereich des Filtermaterials vorhanden ist.

**21.** Verfahren gemäß einem der Ansprüche 15 bis 20, wobei die basischen funktionellen Gruppen tertiäre Aminogruppen sind und die nicht-ionischen hydrophilen Gruppen aus der aus Hydroxylgruppen, Polyethylenoxidgruppen und einem Gemisch davon bestehenden Gruppe ausgewählt sind.

## Revendications

**1.** Matériau filtrant pour séparer des leucocytes d'un produit à base de sang total ou d'un produit à base d'hématies, comprenant au moins une couche d'un élément poreux ayant un réseau tridimensionnel de pores ouverts continus, et dans lequel l'élément poreux a, dans une partie superficielle, des groupes fonctionnels basiques à une densité de $5 \times 10^{-5}$ à $0,1$ mEq/m$^2$ et des groupes hydrophiles non ioniques, un rapport molaire des groupes fonctionnels basiques aux groupes hydrophiles non ioniques étant dans l'intervalle de 0,6 à 6.

**2.** Matériau filtrant suivant la revendication 1, dans lequel l'élément poreux a une valeur de F de 1,6 à 10 g/s·m, la valeur de F étant définie par la formule suivante :

$$\text{Valeur de F} = Q \times R \times 100$$

dans laquelle Q représente la perméabilité à l'air de l'élément poreux (cm$^3$/s·cm$^2$) et R représente le poids de l'élément poreux par centimètre carré (g/cm$^2$), et dans laquelle la perméabilité à l'air est le volume (cm$^3$) de l'air passant dans l'élément poreux par seconde et par centimètre carré de l'élément poreux telle que mesurée à 20°C à une différence de pression de 12,7 mmAq entre les côtés opposés de l'élément poreux conformément à la norme industrielle japonaise (JIS) L-1096.

**3.** Matériau filtrant suivant la revendication 2 ou 3, dans lequel l'élément poreux est choisi dans le groupe consistant en une étoffe fibreuse et une structure poreuse non fibreuse.

**4.** Matériau filtrant suivant la revendication 3, dans lequel l'étoffe fibreuse est une étoffe non tissée comprenant des fibres ayant un diamètre moyen des fibres de 0,3 à 5 µm.

**5.** Matériau filtrant suivant l'une quelconque des revendications 1 à 4, dans lequel les groupes fonctionnels basiques sont des groupes amino tertiaires.

**6.** Matériau filtrant suivant l'une quelconque des revendications 1 à 5, dans lequel les groupes hydrophiles non ioniques sont choisis dans le groupe consistant en groupes hydroxyles, en groupes poly(oxydes d'éthylène) et leur mélange.

**7.** Dispositif filtrant pour séparer des leucocytes d'un produit à base de sang total ou d'un produit à base d'hématies, qui comprend un boîtier ayant un orifice d'entrée du sang et un orifice de sortie du sang, et un matériau filtrant logé dans le boîtier entre l'orifice d'entrée du sang et l'orifice de sortie du sang,

le matériau filtrant comprenant au moins une couche d'un élément poreux ayant un réseau tridimensionnel de pores ouverts continus, et dans lequel l'élément poreux a, dans une partie superficielle, des groupes fonctionnels basiques à une densité de $5 \times 10^{-5}$ à $0,1$ mEq/m$^2$ et des groupes hydrophiles non ioniques, un rapport molaire des groupes fonctionnels basiques aux groupes hydrophiles non ioniques étant dans l'intervalle de 0,6 à 6,
le dispositif filtrant ayant une valeur de P de 3 à 50 mmAq/g, la valeur de P étant définie par la formule suivante :

$$\text{Valeur de P} = \frac{\text{perte de pression de l'air passant dans le dispositif filtrant (mmAq)}}{\text{poids de la surface spécifique de filtration du matériau filtrant (g)}}$$

dans laquelle la perte de pression de l'air passant dans le dispositif filtrant est la différence de pression telle que mesurée entre l'orifice d'entrée du sang et l'orifice de sortie du sang dans des conditions telles que de l'air sec passe dans le dispositif filtrant de l'orifice d'entrée du sang du dispositif à l'orifice de sortie du sang du dispositif à un débit constant de 3 litres par minute à température ambiante.

8. Dispositif filtrant suivant la revendication 7, dans lequel l'élément poreux a une valeur de F de 1,6 à 10 g/s·m, la valeur de F étant définie par la formule suivante :

$$\text{Valeur de F} = Q \times R \times 100$$

dans laquelle Q représente la perméabilité à l'air de l'élément poreux ($cm^3$/s·$cm^2$) et R représente le poids de l'élément poreux par centimètre carré (g/$cm^2$), et dans laquelle la perméabilité à l'air est le volume ($cm^3$) de l'air passant dans l'élément poreux par seconde et par centimètre carré de l'élément poreux telle que mesurée à 20°C à une différence de pression de 12,7 mmAq entre les côtés opposés de l'élément poreux conformément à la norme industrielle japonaise (JIS) L-1096.

9. Dispositif filtrant suivant la revendication 7 ou 8, dans lequel l'élément poreux est choisi dans le groupe consistant en une étoffe fibreuse et une structure poreuse non fibreuse.

10. Dispositif filtrant suivant la revendication 9, dans lequel l'étoffe fibreuse est une étoffe non tissée comprenant des fibres ayant un diamètre moyen des fibres de 0,3 à 5 μm.

11. Dispositif filtrant suivant l'une quelconque des revendications 7 à 10, dans lequel le matériau filtrant a une valeur de S/T de 50 à 300 cm en termes du quotient de la surface spécifique de filtration (S) ($cm^2$) divisée par son épaisseur (T) (cm).

12. Dispositif filtrant suivant l'une quelconque des revendications 7 à 11, dans lequel le matériau filtrant comprend plusieurs couches d'éléments poreux ayant différents diamètres moyens des pores, dans lequel le matériau filtrant a un gradient relativement au diamètre moyen des pores tel que le diamètre moyen des pores diminue sensiblement continuellement ou par degrés d'une partie terminale en amont à une partie terminale en aval du matériau filtrant dans un sens d'écoulement, d'un produit à base de sang total ou d'un produit à base d'hématies, une couche d'un élément poreux qui est présente en amont de la partie terminale en aval du matériau filtrant dans le sens d'écoulement ayant un diamètre moyen des pores qui vaut au moins 1,2 fois celui d'une couche d'un élément poreux présente à la partie terminale en aval du matériau filtrant.

13. Dispositif filtrant suivant l'une quelconque des revendications 7 à 12, dans lequel les groupes fonctionnels basiques sont des groupes amino tertiaires.

14. Dispositif filtrant suivant l'une quelconque des revendications 7 à 13, dans lequel les groupes hydrophiles non ioniques sont choisis dans le groupe consistant en groupes hydroxyles, en groupes poly(oxydes d'éthylène) et leur mélange.

15. Méthode pour séparer des leucocytes d'un produit à base de sang total ou d'un produit à base d'hématies, consistant à soumettre un produit à base de sang total ou d'un produit à base d'hématies à une filtration par un dispositif filtrant,

le dispositif filtrant comprenant un boîtier ayant un orifice d'entrée du sang et un orifice de sortie du sang, et un matériau filtrant logé dans le boîtier entre l'orifice d'entrée du sang et l'orifice de sortie du sang,
le matériau filtrant comprenant au moins une couche d'un élément poreux ayant un réseau tridimensionnel de pores ouverts continus, et dans lequel l'élément poreux a, dans une partie superficielle, des groupes fonctionnels basiques à une densité de $5 \times 10^{-5}$ à 0,1 mEq/$m^2$ et des groupes hydrophiles non ioniques, un rapport molaire des groupes fonctionnels basiques aux groupes hydrophiles non ioniques étant dans l'intervalle de 0,6 à 6,
Le dispositif filtrant ayant une valeur de P de 3 à 50 mmAq/g, la valeur de P étant définie par la formule suivante :

$$\text{Valeur de P} = \frac{\text{perte de pression de l'air passant dans le dispositif filtrant (mmAq)}}{\text{poids de la surface spécifique de filtration du matériau filtrant (g)}}$$

dans laquelle la perte de pression de l'air passant dans le dispositif filtrant est la différence de pression telle que mesurée entre l'orifice d'entrée du sang et l'orifice de sortie du sang dans des conditions telles que de l'air sec passe dans le dispositif filtrant de l'orifice d'entrée du sang du dispositif à l'orifice de sortie du sang du dispositif à un débit constant de 3 litres par minute à température ambiante,

le produit à base de sang total ou le produit à base d'hématies étant traité en une quantité de 0,1 à 0,5 unité par gramme du matériau filtrant.

16. Méthode suivant la revendication 15, dans laquelle l'élément poreux a une valeur de F de 1,6 à 10 g/s · m, la valeur de F étant définie par la formule suivante :

$$\text{Valeur de F} = Q \times R \times 100$$

dans laquelle Q représente la perméabilité à l'air de l'élément poreux ($cm^3$/s · $cm^2$) et R représente le poids de l'élément poreux par centimètre carré ($g/cm^2$), et dans laquelle la perméabilité à l'air est le volume ($cm^3$) de l'air passant dans l'élément poreux par seconde et par centimètre carré de l'élément poreux telle que mesurée à 20°C à une différence de pression de 12,7 mmAq entre les côtés opposés de l'élément poreux conformément à la norme industrielle japonaise (JIS) L-1096.

17. Méthode suivant la revendication 15 ou 16, dans laquelle l'élément poreux est choisi dans le groupe consistant en une étoffe fibreuse et une structure poreuse non fibreuse.

18. Méthode suivant la revendication 17, dans laquelle l'étoffe fibreuse est une étoffe non tissée comprenant des fibres ayant un diamètre moyen des fibres de 0,3 à 5 $\mu$m.

19. Méthode suivant l'une quelconque des revendications 15 à 18, dans laquelle le matériau filtrant a une valeur de S/T de 50 à 300 cm en termes du quotient de la surface spécifique de filtration (S) ($cm^2$) divisée par son épaisseur (T) (cm).

20. Méthode suivant l'une quelconque des revendications 15 à 19, dans laquelle le matériau filtrant comprend plusieurs couches d'éléments poreux ayant différents diamètres moyens des pores, dans laquelle le matériau filtrant a un gradient relativement au diamètre moyen des pores tel que le diamètre moyen des pores diminue sensiblement continuellement ou par degrés d'une partie terminale en amont à une partie terminale en aval du matériau filtrant dans un sens d'écoulement, d'un produit à base de sang total ou d'un produit à base d'hématies, une couche d'un élément poreux qui est présente en amont de la partie terminale en aval du matériau filtrant dans le sens d'écoulement ayant un diamètre moyen des pores qui vaut au moins 1,2 fois celui d'une couche d'un élément poreux présente à la partie terminale en aval du matériau filtrant.

21. Méthode suivant l'une quelconque des revendications 15 à 20, dans laquelle les groupes fonctionnels basiques sont des groupes amino tertiaires et les groupes hydrophiles non ioniques sont choisis dans le groupe consistant en groupes hydroxyles, en groupes poly(oxydes d'éthylène) et leur mélange.